# EUROPEAN PATENT SPECIFICATION

(11) **EP 2 793 962 B1**
(45) Date of publication and mention of the grant of the patent: **27.04.2016**
(21) Application number: 12813261.0
(22) Date of filing: 21.12.2012
(51) Int. Cl.: A61L 27/34, A61L 29/08, A61L 31/10, A61L 27/50, A61L 29/14, A61L 31/14

(54) **PROCESS FOR MODIFYING THE SURFACE MORPHOLOGY OF A MEDICAL DEVICE**
VERFAHREN ZUR MODIFIZIERUNG DER OBERFLÄCHENMORPHOLOGIE EINER MEDIZINISCHEN VORRICHTUNG
PROCÉDÉ DE MODIFICATION DE LA MORPHOLOGIE DE SURFACE D'UN DISPOSITIF MÉDICAL

(30) Priority: 23.12.2011 DK 201170752
(43) Date of publication of application: 29.10.2014
(73) Proprietor: Le, Dang Quang Svend, 8000 Aarhus C (DK); Chen, Muwan, 8000 Aarhus C (DK); Bünger, Cody Eric, 8963 Auning (DK); Besenbacher, Flemming, 8210 Aarhus V (DK)
(72) Inventor: Le, Dang Quang Svend, 8000 Aarhus C (DK); Chen, Muwan, 8000 Aarhus (DK); Chen, Menglin, 8240 Risskov (DK); Baatrup, Anette Overgaard, 8240 Risskov (DK); Bünger, Cody Eric, 8963 Auning (DK); Lysdahl, Helle, 8320 Marslet (DK); Kjems, Jorgen, 8240 Risskov (DK); Andersen, Morten Ostergaard, 5000 Odense C (DK); Besenbacher, Flemming, 8210 Aarhus V (DK)
(74) Representative: Plougmann Vingtoft a/s
(86) International application number: PCT/DK2012/050507
(87) International publication number: WO 2013/091662

(56) References cited:
- EP-A1- 2 266 635
- US-A- 5 527 337
- US-A1- 2007 286 941
- US-A1- 2008 305 141
- US-A1- 2009 148 591
- US-B1- 6 767 928

## Description

### Technical field of the invention

The present invention relates to the field of medical devices. In particular, the present invention relates to a process for modifying the surface morphology of a medical device and the products derived therefrom.

### Background of the invention

Controlling the performance of medical devices, for example controlling the release of drugs, is an important aspect in medical device design. The performance is in part dictated by the size of the device surface area and its specific physical properties such as stiffness and topography. For example, an increased surface area on a medical device affects drug adsorption speed and capacity, the drug release rate, cell adherence and cellular phenotypes such as stem cell differentiation. A modified surface can be produced by various methods including etching and coating. However, both these methods have drawbacks. Etching has the drawback of breaking down the molecules in the surface layer while introducing new surface chemistry. These issues complicate the prediction of surface properties and may lead to undesired detrimental effects. Furthermore, the coating of a medical device results in a complex multicomponent system. In the latter case, polymer components are selected such that the mixture or composition meets the desired characteristics based on their individual characteristics. Unfortunately, the combinations do not always exhibit a combination of the characteristics present in the individual components. Polymer characteristics vary from polymer to polymer based on a host of factors that can depend on the chain-to-chain interaction in the polymer or the arrangement of functional groups at the polymer's surface, among other factors. But when one polymer is combined with another, some of the factors are no longer present. Using the example above, the chain-to-chain interaction in a polymer combination can differ because the types of chains differ. Similarly, the arrangement of functional groups at the surface can be different because the mixture contains a different cohort of surface groups.

A common type of medical device is the macroporous scaffold. The internal surfaces of these can be modified by filling the macropores of the top down controlled architecture with a second microporous structure generated by phase separation. While these methods increase the surface area of the scaffold, they also disrupt the original pore interconnectivity which can limit cell penetration, nutrient transport, and, most importantly, drug loading into the scaffold. Another method is the fabrication of a highly porous FDM by leaching out watersoluble PEO porogens that are coextruded with the scaffold polymer proper. FDM using polymer solution rather than polymer melt has also been reported to produce increased porosity without obliterating macroporous space. Accelerated hydrolytic degradation is also a possibility to increase surface area and porosity. These methods, however, arguably compromise the mechanical load-bearing capabilities normally associated with FDM scaffolds.

WO2007/106247 discloses a method wherein a solvent + a polymer is used to form a coating of the polymer on e.g. a stent by phase inversion, thereby forming a layer of the coating on the medical device.

US 2007/286941 discloses methods of treating a polymeric surface of a stent by contacting the surface with a primary solvent, and subsequently contacting the surface with a secondary solvent - which is a non-solvent for the polymer - to remove the first solvents.

US 5527337 shows methods for forming pores on the surface of a biodegradable stent by contacting the stent with a solvent and a non-solvent for the stent material. The resulting porous area of the stent can be filled with drugs and/or allow both blood flow and tissue ingrowth.

US 2008/305141 discloses methods of producing a medical device having a coating with a controlled morphology and thickness which involves: preparing a coating composition comprising a polymer, a solvent and a therapeutic agent, applying the coating composition onto a medical device to form a wet coating layer, and subjecting the wet coating layer to a freeze-thaw cycle. The wet coating can be dried by removing the solvent from the coating layer with a non-solvent.

US 2009/148591 describes methods to improve adhesion of polymer coatings over polymer surfaces of stents, which include surface treatments with solvents / non-solvents.

EP 2266635 discloses manufacturing methods for three-dimensional biocompatible scaffolds. The porous scaffolds obtained are subjected to a hydrolytic treatment and have a granule size of some millimeters.

Furthermore, medical devices change surface morphologically during processing and storage, as well as after application in vivo. Hence, the predictability, and consequently the clinical utility, of a medical device may rely on the ability to control these changes.

Accordingly, there is a need for control of the surface morphology of a medical device comprising a polymeric composition in the surface region.

### Summary of the invention

The present invention provides a method for modifying the surface morphology of a medical device for controlling the release of an agent from the surface region. The invention can also find use in bioproduction where the same qualities with respect to cell attachment, proliferation, and biocompatibility are advantageous for generation of cellular products from adherent cells.

Thus, one aspect of the present invention relates to a method for modifying a surface morphology of a medical device comprising:
a) providing a medical device comprising a bulk part and a surface region, said surface region comprises a biocompatible polymeric composition comprising a biocompatible polymer;
b) contacting at least a portion of the biocompatible polymeric composition in said surface region with a solvent of the biocompatible polymer to form a first surface region film on at least a first part of said surface region;
c) contacting at least a part of said first surface region film with a non-solvent of said biocompatible polymer, thereby forming a first solidified surface region film on at least a part of said surface region; and
d) subjecting the first solidified surface region film to a hydrolytic treatment.

Another aspect of the present invention relates to a medical device obtainable by the method of the invention.

### Brief description of the figures

Figure 1 shows a possible setup of the method of the present invention. A base scaffold is wetted with a solvent, and left exposed to said solvent for a period of time after which the scaffold is immersed in a coagulation bath comprising a non-solvent,
Figure 2 shows a SEM image of an unmodified FDM PCL scaffold that present little surface structuring on the fiber surface. The dimples mark the borders between individual PCL spherulites, which form during cooling of the polymer melt. 400 x magnification,
Figure 3 shows representative environmental SEM images arranged according to table 2. Marked differences are discernible. 800x magnification,

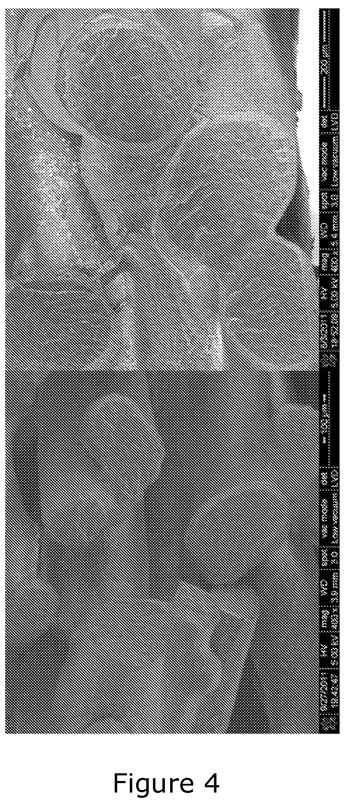

Figure 4 shows: Left: FDM scaffold built with 100 µm thick fibers and a center-center fiber distance of 500 µm thickness and a layer thickness of 80 µm (100/0, 1s, 70/30). Reduction of interconnectivity was successfully avoided by reducing solvent exposure time. Right: Cell culture treated scaffold for size comparison. 400x magnification,

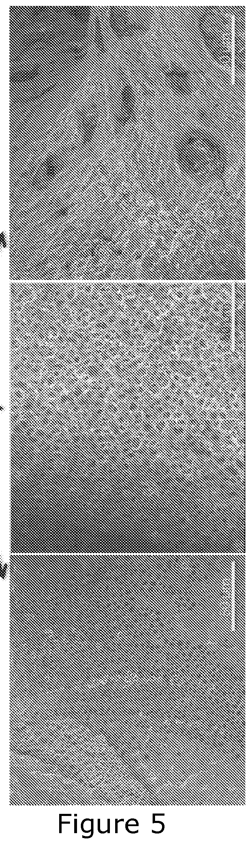

Figure 5 shows: Left: Mitigation of the non-solvent gradient at the phase separation interface by using a non-solvent that is a mixture comprising a pure non-solvent, ethanol, and pure solvent, dioxane, at appropriate fractions dramatically changes the appearance of the surface from the typical cavernous/vacuolar to more disordered surface with smaller features. Top) Solvent bath consisting of 100% dioxane and a non-solvent bath of 70% ethanol and 30% water. Middle) Solvent bath consisting of 100% dioxane and a non-solvent bath of 90% ethanol and 10% dioxane. Bottom) Solvent bath consisting of 100% dioxane and a non-solvent bath of 80% ethanol and 20% dioxane.
Figure 6 shows a device for flushing scaffolds with solvent or non-solvent comprising a funnel that tightly fits over the scaffold. Solvent and non-solvent is admitted through the top inlet. Inside vanes are present to direct fluid flow.

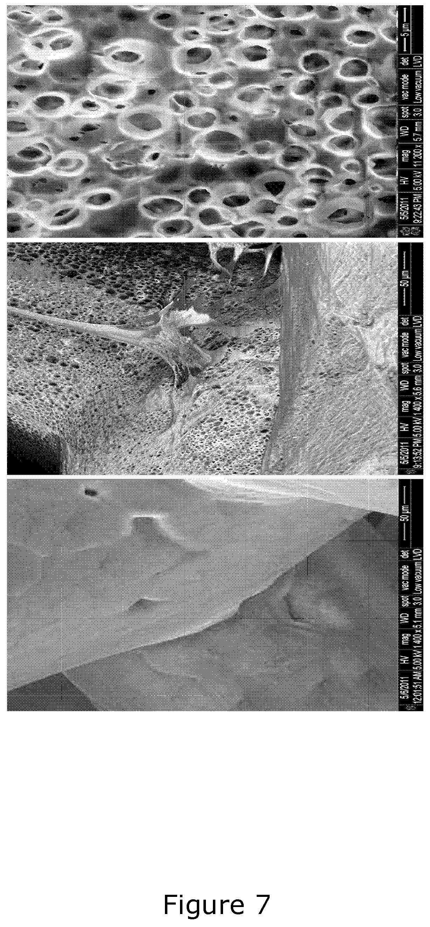

Figure 7 shows surface morphologies of the scaffolds. Left: Appearance of a NaOH treated control scaffold at a fiber junction. The nearly smooth fiber surface features polygonal approximately 50 µm wide spherulite domains with dimples located at their borders (1400x). Middle: Treated (solvent-non-solvent treatment; the term "PIPA" is also used for the surface treatment according to the present invention) scaffold at same magnification showing a substantially higher degree of surface structure. The morphology of the adherent cell central in the image is highly spread out (1400x) Right: PIPA surface at higher magnification showing the porous features (11200x).

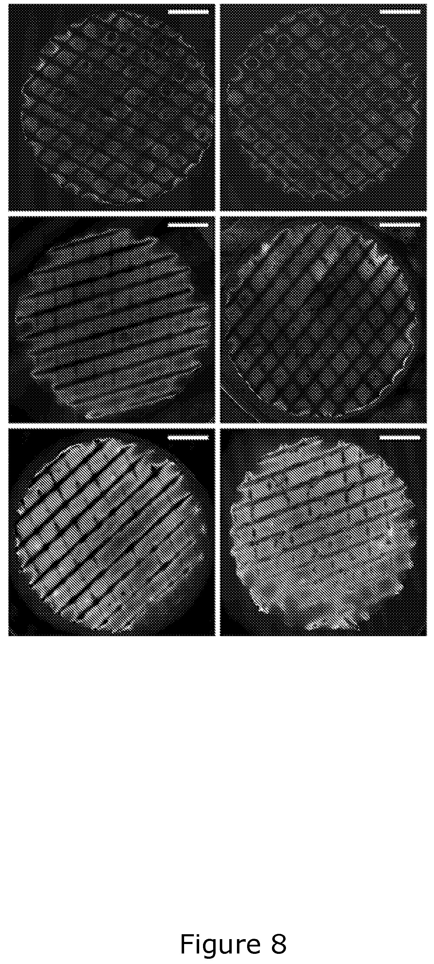

Figure 8 shows CellTracker stained confocal micrograph showing the upper face of the scaffolds at day 2+7 (top row), 2+14 (middle), and day 2+21 (bottom). The pores are completely covered by a confluent cell sheet for both PCL (left column) and treated scaffolds (right column) at day 2+14.

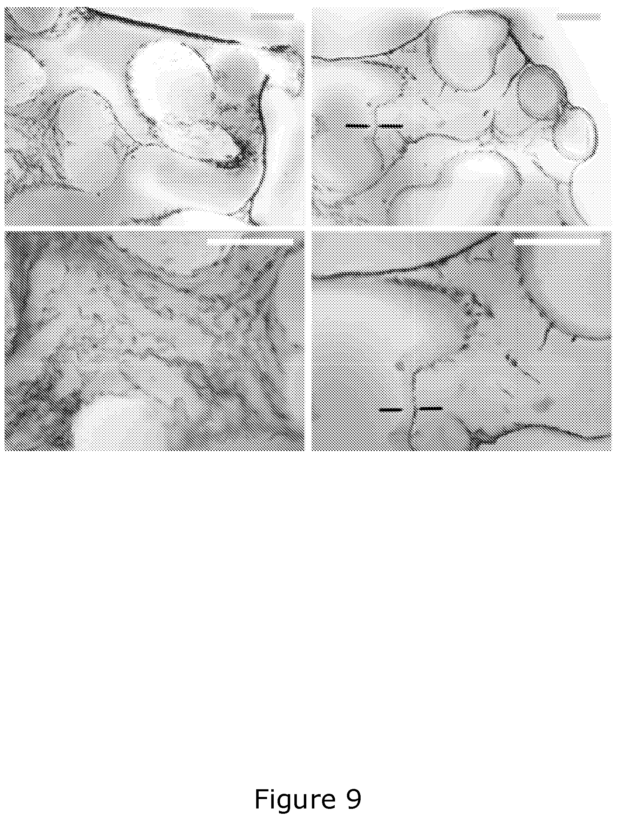

Figure 9 shows the distribution of cells inside the upper peripheral part of the scaffolds at day 2+21 in DEXA media. Top left: Statically cultured control scaffold with a dense intensely stained cell sheet. Fibrous matrix deposition can be seen in in the left part of the image. Bottom left: Higher magnification micrograph of the control scaffold. The cells have a spindle-like fibroblastic appearance. Top right: Treated (PIPA) scaffold with the characteristic lightly stained porous border (arrows) between the solid fiber core and the adherent cells. The apparent variation in border thickness comes from the changing angles between the section plane and the fibers. Bottom right: Semi-cuboidal basophilic cells are sparsely present.
Figure 10 shows DNA amount per scaffold. Cells were seeded on the scaffolds with or without surface modification (PIPA) and were cultivated under static conditions for 2+7, 2+14, 2+21 days, and 2+21 days with DEXA from day 2+14. The amount is expressed as mean ± SD (n=3). *Significant difference between scaffolds within the same time point (P<0.05),
Figure 11 shows ALP activity normalized by µg DNA per scaffold. Cells were seeded on the scaffolds with or without surface modification (PIPA) and were cultivated under static conditions for 2+7, 2+14, 2+21 days, and 2+21 days with DEXA from day 2+14. The activity is expressed as mean ± SD (n=3). Activity is indicated in nanomole p-nitrophenol/microgram DNA per minute (nmol/µg DNA*min). *Significant difference between scaffolds within same time point (P<0.05).
Figure 12 shows Calcium content per scaffold normalized to DNA content on 2+21 after 7 days of culture in DEXA. The amount of calcium is expressed as mean ± SD (n=4), (P<0.05).
Figure 13 shows cumulative release kinetics for three different transfection reagents, Oligofectamine (top), Lipofectamine2000 (middle), TransIT-TKO (bottom) from PCL+H2O, PCL+NaOH, PIPA+H2O, and PIPA+NaOH. PIPA scaffolds showed slower release over the 4 week period.
Figure 14 shows cumulative release kinetics for bovine serum albumin (left) and lysozyme (right) from PCL+H2O, PCL+NaOH, PIPA+H2O, and PIPA+NaOH. PIPA+NaOH scaffolds showed slower release over the 4 week period.
Figure 15 shows the cumulative release at day 28. The darker 4 week release bar is comparable with data from figure 13. The residual accounts for the protein amount that trapped inside the scaffold pores and not yet diffused out into the surrounding media.
Figure 16 shows how it is possible to control scaffold stiffness through macroporous geometry. Left) A PIPA scaffold with a diameter of 4 mm and a height of 2 mm. The direction of the fused deposition modeled fibers changes with 105 degrees pr layer. Right) An example of a scaffold where fiber direction in every second layer is angled by 60 degrees.
Figure 17 shows mechanical test results indicating that the mechanical stiffness for a scaffold is highly anisotropic with the highly anisotropic geometry shown in figure 16.
Figure 18 shows cumulative release curves for doxorubicin adsorbed onto PCL scaffolds. The release kinetic is dependent on loading amount, hydrolytic treatment, and morphology.
Figure 19 shows stained sections of PIPA scaffolds following implantation in a pig dorsal muscle pocket. A) Unloaded PIPA_A+NaOH scaffold shown in its entirety with surrounding muscle B) Drug loaded PIPA_A+NaOH scaffold with necrotic cells in the pore space C+D) Close up of the unloaded scaffold with voids indicating the rapid prototyped scaffold. The PIPA modification has a dark stain (arrow). D+F) The partially drug loaded scaffold is filled with dead or multinucleated cells. Bars are 1 mm, 500 µm, and 200 µm, respectively.
Figure 20 shows the incorporation of fluorescent nanoparticles in the scaffold. A) Control PIPA scaffold without incorporation of particles viewed in a fluorescence microscope (white light without barrier filter). B) Absence of a fluorescent signal at excitation at 528 nm and a barrier filter of 644 nm. C) Particle incorporated scaffold. D) Presence of a fluorescent signal. Bars are 200 µm.

The present invention will now be described in more detail in the following.

### Detailed description of the invention

The present invention relates to a process for modifying the surface morphology of a medical device to obtain an improved surface for cell attachment and/or bioactive substance release.

One aspect of the invention relates to a method for modifying a surface morphology of a medical device comprising:
a) providing a medical device comprising a bulk part and a surface region, said surface region comprises a biocompatible polymeric composition comprising a biocompatible polymer;
b) contacting at least a portion of the biocompatible polymeric composition in said surface region with a solvent of the biocompatible polymer to form a first surface region film on at least a first part of said surface region;
c) contacting at least a part of said first surface region film with a non-solvent of said biocompatible polymer, thereby forming a first solidified surface region film on at least a part of said surface region; and
d) subjecting the first solidified surface region film to a hydrolytic treatment.

In an embodiment the said non-solvent may comprise a second dissolved polymer for which the said non-solvent is a solvent. Said second polymer can be solidified by contacting the at least a part of surface region with a second non-solvent for the second polymer.

As used herein, the term 'solvent' refers to a solvent of a polymer in the biocompatible polymeric composition of the surface region. The term 'non-solvent' refers to a non-solvent of a polymer in the biocompatible polymeric composition of the surface region. Hence, the choices of solvents and non-solvents are polymer dependent and the solvent for one set of polymers may act as non-solvent for a different set of polymers.

Suitable solvents include, but are not limited to, for example, CH₂Cl₂, chloroform, ethanol, isopropanol, n-propanol, dimethylformamide, dimethylacetamide, and dimethylsulfoxide. In some embodiments, the solvent can be alcohols (e.g., methanol, ethanol, 1,3-propanol, 1,4-butanol), heptane, hexane, pentane, cyclohexanone, trichloro ethane, acetone, tetrahydrofuran (THF), dimethyl acetamide (DMAc), dioxane, toluene, xylene, dimethyl sulfoxide (DMSO), dimethyl formamide (DMF), ethyl acetate, methyl ethyl ketone (MEK), and acetonitrile.

Suitable non-solvents include, but are not limited to, for example, water, dioxane, acetone, toluene, cyclohexanone, methylethyl ketone, benzene, toluene, xylene, pentane, hexane, cyclohexane, octane.. Table 1 summarizes some examples of solvents or non-solvents of a specific polymer.

| Polymer | Solvent | Non-solvent |
|---|---|---|
| PLLA, PDLA, PLGA, PLCL, polystyrene | Cresol, 1,4-dioxane, chloroform, tetrahydrofuran, Dichloromethane, Acetone, N-methyl pyrrolidone | H₂O, Methanol, Ethanol, Isopropanol, Glycerol |
| PCL | Cresol, 1,4 dioxane, chloroform, tetrahydrofuran, dichloromethane | H₂O, Methanol, Ethanol, Isopropanol, Glycerol, Acetone, Dimethyl sulphoxide, Dimethyl formamide, cyclohexane, methyl cyclohexane, diisoutylene, ethane diol, digol, |
| PGA | Hexafluoropropanol | H₂O, Methanol, Ethanol, Isopropanol, Glycerol, Acetone, |
| Hyaluronic acid | H₂O | Ethanol |

The invention also allows for but is not limited to binary, ternary solvent systems, with which it is possible to substitute expensive, harmful, potentially environmentally harmful solvents with solvent mixtures comprising cheaper and more benign solvent components. One example is the use of a blend of ethanol, water, and calcium chloride rather than hexafluoroisopropanol to dissolve silk fibroin.

The use of mixed solvents and mixed non-solvents in the invention is also advantageous for controlling the liquids' volatilities, solvation powers, viscosities, surface tensions, densities, dielectric strengths, melting and boiling point, miscibility properties, temperature-dependent solvation powers, thermal conductivities and contraction rates. These variables can be varied to create a vast variation in generated surface morphology for a wide range of polymer devices.

Below is a table showing different solvent mixtures which may be used for the indicated polymers.

| Polymer | Solvent mixtures | Non-solvent |
|---|---|---|
| PLLA, PDLA, PLGA, PLCL, polystyrene | Cresol 1,4-dioxane 50%:50%, 1,4-dioxane chloroform 40%: 50%, 1,4-dioxane chloroform 90%: 10%, tetrahydrofuran dichloromethane 80%:20%, dioxane acetone 10%:90%, N-methyl pyrrolidone acetone 23%:77%, dioxane water 98%:2%, dioxane water 87%:13%, dioxane ethanol 87%:13%, dioxane chloroform ethanol 90%:5%:5%, acetone ethanol 98%:2%, acetone ethanol 70%:30% | H₂O Methanol 90%:10%, H₂O Ethanol 30%:70%, H₂O Ethanol 10%:90%, H₂O Isopropanol 25%:75%, H₂O Glycerol 5%:95%, Ethanol glycerol 50%:50%, Ethanol glycerol isopropanol 20%:40%: 40%, Ethanol dioxane 90%:10%, glycerol dioxane 95%:5%, glycerol isopropanol acetone 40%:45%: 10% |
| PCL | Cresol 1,4-dioxane 50%:50%, 1,4-dioxane chloroform 40%: 50%, 1,4-dioxane chloroform 90%: 10%, tetrahydrofuran dichloromethane 80%:20%, dioxane acetone 90%:10%, N-methyl pyrrolidone | H₂O Methanol 90%:10%, H₂O Ethanol 30%:70%, H₂O Ethanol 10%:90%, H₂O Isopropanol 25%:75%, H₂O Glycerol 5%:95%, Ethanol glycerol 50%:50%, Ethanol glycerol isopropanol 20%:40%: 40%, Ethanol dioxane |
| | acetone 23%:77%, dioxane water 98%:2%, dioxane water 87%:13%, dioxane ethanol 87%:13%, dioxane chloroform ethanol 90%:5%:5%, xylene/isopropanol 90%:10%, xylene/isopropanol 80%:20%, xylene/butanol 95%:5%, xylene butanol 90%:10% xylene butanol 80%:10%, dioxane glycerol 95%:5% | 90%: 10%, glycerol dioxane 95%:5%, glycerol isopropanol acetone 40%:45%: 10%, H₂O aceton 90%:10%, H₂O DMSO 30%:70%, Ethanol acetone 90%:10%, H₂O Isopropanol |
| PGA | Hexafluoropropanol | H₂O, Methanol, Ethanol, Isopropanol, Glycerol, Acetone, |
| Hyaluronic acid | H₂O | Ethanol |

The term 'biocompatible' is to be understood as, but not limited to, eliciting little or no immune response in a given organism. Indeed, since the immune response and repair functions in the body are highly complicated, it is not adequate to describe the biocompatibility of a single material in relation to a single cell type or tissue.

As previously mentioned WO 2007/106247 discloses a method wherein a solvent + a polymer is used to form a coating of the polymer on e.g. a stent by phase inversion, thereby forming a layer of the coating on the medical device. WO 2007/106247 is silent in respect of the biocompatible polymer being present in the bulk part of the medical device before initiating the process. Instead the polymer is present in the solvent. Thus, the polymer is an addition to the original medical device thereby providing a medical device with an increased density and volume. This may be a disadvantage for porous structures e.g. microporous structures, since the addition of material may remove/fill out the pores and decrease interconnectivity, thereby destroying the structure and limit the applicability of the device.

Furthermore, as polymer solutions always have a higher viscosity than the pure solvent, it will prove difficult to rapidly and homogeneously permeate a large scaffold with small pores with a solution due to high fluid resistance.
On the other hand, the method of the present invention exploits the presence of the biocompatible polymer in the medical device to rearrange the surface structure of the exposed part of the medical device in a controlled manner (providing the first solidified surface region film). Thus, the method of the invention may be performed in the absence of polymer in the solvent.

The use of a solvent rather than a polymer solution is furthermore advantageous because it among other things allows the process to involve temperatures that are lower than the lowest critical solution temperature of a given polymer solution, which could cause the a polymer solution to phase separate into polymer-rich and -poor regions out of contact with the device surface - thus decreasing the probability of a desired homogenous morphology modification throughout the device's pores. Furthermore, using a solvent rather than a polymer solution is advantageous as it allows for thermal control of solvation power.
In one advantageous embodiment, a surface region of a highly complex geometry is wetted with a cold yet liquid solvent with decreased solvation power. The surface region itself can have been uniformly or ununiformly pre-cooled or - heated, or remain at ambient temperature prior to wetting. As the solvent has wetted the entirety of the surface region, the all of the surface region or parts thereof can be cooled or heated to globally or locally decrease or increase solvation power and thus the degree of surface modification. This is an advantage for making e.g. biphasic scaffolds for e.g. cartilage repair.

In an embodiment the provided solvent does not comprise the biocompatible polymer dissolved therein. In yet an embodiment the solid content in the provided solvent of the biocompatible polymer is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%. To obtain the optimal surface modification the solid content is preferably kept low. It is also preferred that the provided solvent does not comprise the biocompatible polymer dissolved therein, however other solids may be present. Thus, in a further embodiment the solid content in the provided solvent is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%.

In yet another embodiment, the solvent of the biocompatible polymer comprises a second biocompatible synthetic or natural polymer. Such polymer may be incorporated in the medical device.

In another advantageous embodiment, the temperature-controlled solvent contains a synthetic prepolymer such as a polyol and the temperature-controlled non-solvent contains an activator or a crosslinker such as an isocyanate or examples from the non-exhaustive list: Adipic acid dihydrazide, 5-Azido-2-nitrobenzoic acid N-hydroxysuccinimide ester, 6-(4-Azido-2-nitrophenylamino)hexanoic acid N-hydroxysuccinimide ester, 4-Azidophenacyl bromide, Benzophenone-4-isothiocyanate, (1R,8S,9s)-Bicyclo[6.1.0]non-4-yn-9-ylmethyl N-succinimidyl carbonate, Bis[2-(4-azidosalicylamido)ethyl] disulfide, Bis(polyethylene glycol bis[imidazoyl carbonyl]), 1,4-Bis[3-(2-pyridyldithio)propionamido]butane, Bis[2-(N-succinimidyl-oxycarbonyloxy)ethyl] sulfone, O,O'-Bis[2-(N-Succinimidyl-succinylamino)ethyl]polyethylene glycol, Bromoacetic acid N-hydroxysuccinimide ester, Dibenzocyclooctyne-S-S-N-hydroxysuccinimidyl ester, Dibenzocyclooctyne-maleimide, Dibenzocyclooctyne-PEG4-maleimide, 4,4'-Diisothiocyanatostilbene-2,2'-disulfonic acid, Dimethyl 3,3'-dithiopropionimidate dihydrochloride, Dimethyl pimelimidate dihydrochloride, Di(N-succinimidyl) glutarate, 3,3'-Dithiodipropionic acid di(N-hydroxysuccinimide ester), Ethylene glycol-bis(succinic acid N-hydroxysuccinimide ester), Ethylene glycol disuccinate bis(sulfo-N-succinimidyl) ester, 4,7,10,13,16,19,22,25,32,35,38,41,44,47,50,53-Hexadecaoxa-28,29-dithiahexapentacontanedioic acid di-N-succinimidyl ester, Iodoacetic acid N-hydroxysuccinimide ester, Maleimidoacetic acid N-hydroxysuccinimide ester, 3-Maleimidobenzoic acid N-hydroxysuccinimide ester, 4-(N-Maleimido)benzophenone, 4-Maleimidobutyric acid N-hydroxysuccinimide ester, 6-Maleimidohexanoic acid N-hydroxysuccinimide ester, 4-(N-Maleimidomethyl)cyclohexanecarboxylic acid N-hydroxysuccinimide ester, 4-(N-Maleimidomethyl)cyclohexane-1-carboxylic acid 3-sulfo-N-hydroxysuccinimide ester, 4-(4-Maleimidophenyl)butyric acid N-hydroxysuccinimide ester, 3-(Maleimido)propionic acid N-hydroxysuccinimide ester, 3-Maleimidopropionic acid N-hydroxysuccinimide ester, O-[N-(3-Maleimidopropionyl)aminoethyl]-O'-[3-(N-succinimidyloxy)-3-oxopropyl]heptacosaethylene glycol, O-[N-(3-Maleimidopropionyl)aminoethyl]-O'-[3-(N-succinimidyloxy)-3-oxopropyl]triethylene glycol, 4-(Maleinimido)phenyl isocyanate, 4,4'-Methylenebis(phenyl isocyanate), p-Phenylene diisothiocyanate, 3-(2-Pyridyldithio)propionic acid N-hydroxysuccinimide ester, Sebacic acid bis(N-succinimidyl) ester, Suberic acid bis(N-hydroxysuccinimide ester), Suberic acid bis(3-sulfo-N-hydroxysuccinimide ester), Sulfo-dibenzocyclooctyne-biotin, Sulfo-N-succinimidyl 4-maleimidobutyrate, Trimethylolethane triglycidyl ether.
The non-solvent may also comprise a catalyst. It is thus possible to achieve prepolymer polymerization on the surface region.

In another advantageous embodiment, the solvent comprises the crosslinker and the non-solvent comprises the prepolymer.

In yet another advantageous embodiment, the crosslinker is photosensitive and the device is partially or completely irradiated following introduction of the crosslinker.

In yet another embodiment, the solvent contains a polymer the non-solvent contains a crosslinker such as genipin, formaldehyde, Ca²⁺, peroxides, or cinnamaldehyde. The advantage of the crosslinker is to improve adhesion to the medical device.

In another advantageous embodiment, the solvent contains a biological extracellular matrix component such as a collagen or a collagen precursor or derivative and the non-solvent contains a crosslinker such as genipin.

It is also to be understood that the solvent may be a mixture of different solvents. In an embodiment the solvent is a mixture of more than one type of solvent. IN yet an embodiment the mixture of solvents is a mixture of 1-5 solvents, such as 1-4, such as 1-3, such as 1-2, such as 2 or 3. In a more specific example the the mixture of solvents for e.g. PCL under cold conditions include toluene/DMSO, xylene/isopropanol 90%:10%, xylene/isopropanol 80%:20%, xylene/butanol 95%:5%, xylene butanol 90%:10% xylene butanol 80%:10%, and dioxane glycerol 95%:5%.

Similarly, the non-solvent may also be a mixture of non-solvents. Thus, in an embodiment the non-solvent is a mixture of more than one type of non-solvents. In yet an embodiment the mixture of non-solvents is a mixture of 1-5 non-solvents, such as 1-4, such as 1-3, such as 1-2, such as 2 or 3. In a more specific example the mixture of non-solvents for PCL is EtOH/water. Mixtures of at least 1 non-solvent are advantageous because they allow modification solvation powers, viscosities, surface tensions, densities, melting point, miscibility properties, temperature-dependent solvation powers, thermal diffusivities and thermal contraction rates. These variables can be varied to create a vast variation in generated surface morphology for a wide range of polymer devices.

A mixture may still qualify as a non-solvent even though it comprises partly a solvent. Thus, in an embodiment the non-solvent is a mixture of a non-solvent and a solvent. Partial addition of a solvent helps to control i.e. flatten the solvent concentration gradient over the formed solution film which decreases the solvent transfer rate from the solution film to the non-solvent mixture and thus the speed of phase separation. Among other things, this is beneficial for the formation of a open surface morphology.

A mixture comprising pure non-solvent components and pure solvent components qualifies as a non-solvent for a polymer under a given set of processing conditions if in it the polymer is insoluble. Depending on the solvent resistance of the polymer a very large variety of mixtures can be devised. With some exceptions, e.g. Polyethylene glycol and polyvinylalcohol, most common synthetic aliphatic polymers are insoluble in water and many are insoluble in ethanol, isopropanol, glycerol, methanol and others. Thus it is advantageous to devise non-solvent mixtures where these substances form the largest fraction. The type and fraction size of the solvent to be added to the mixture depends on the solvent resistance of the composition of polymers that make up the surface

In a further embodiment the mixture of a non-solvent and a solvent is, a non-exhaustive list for a surface region that comprises PCL of non-solvent mixtures that comprise solvents includes: Water dioxane 90%;10%, ethanol toluene 85% 15%, glycerol tetrahydrofuran 95 %: 5%, Water ethanol glycerol dioxane tetrahydrofuran 40%: 10% : 50% : 5% : 5%.
Preferably the entire medical device comprises the biocompatible polymeric composition comprising a biocompatible polymer and furthermore the entire medical device is subjected to the method of the invention. Thus, in an embodiment the entire medical device comprises the biocompatible polymeric composition comprising a biocompatible polymer on the surface and wherein the entire medical device is subjected to the method steps b) and c).

The medical device may also be completely or substantially completely be composed of biocompatible polymer (e.g. PCL). Thus, in yet a further embodiment the medical device consists of or substantially consists of the biocompatible polymeric composition comprising a biocompatible polymer. This is a clear advantage of biodegradable medical devices is intended. Furthermore it makes the production much simpler, since e.g. a subsequent coating with the polymer is avoided.

In one embodiment of the present invention, the biocompatible polymeric composition is biodegradable.

The term 'biodegradable' is to be understood as, but not limited to, the chemical, such as biochemical, breakdown of materials by a physiological environment, such as in a human or animal.

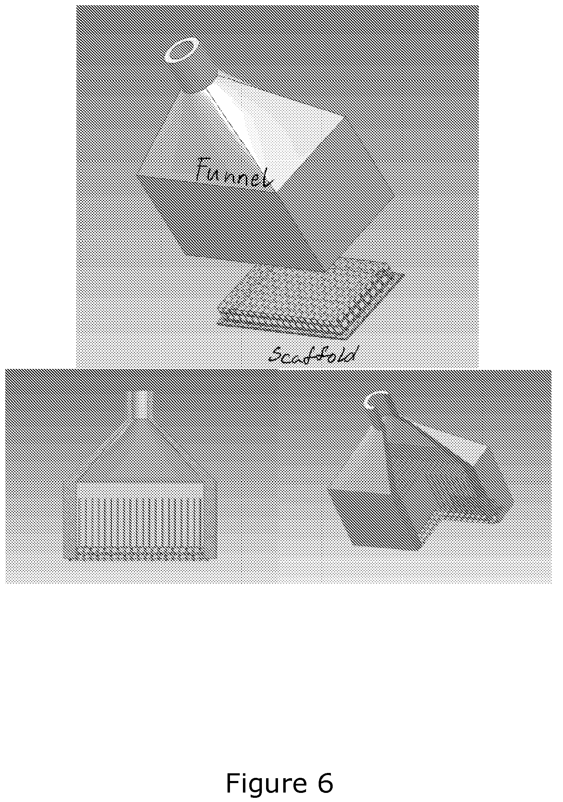

The solidified surface region film formed on the surfaces, or part of the surfaces, of the medical device is thought to be generated by redistribution/phase separation of the surface region film. First, a film of polymer solution is formed on the surface area that has been into contact with the solvent, e.g. by soaking the medical device in a solvent of the biocompatible polymeric composition and dissolving the wetted surfaces. In the case of a tissue scaffold, the macro-porous geometry facilitates efficient and even wetting by the solvent through capillary forces. Then, immersion of the medical device, now comprising a surface region film, into a non-solvent, e.g. a coagulation bath, both purges the large pores of excess polymer solution and extracts the solvent from the film thereby precipitating a solid surface region film. An alternative way of performing such operation could be by use of the device disclosed in figure 6. Here, the solvent and non-solvent can be forced through the pores of a medical device, such as a scaffold or a stent.

In one embodiment of the present invention, the contact of the solvent with at least a part of the first surface region film can be done by condensation of a vapour phase of the solvent onto a part of the first surface region. This can be done by lowering the temperature of the vapour or by increasing the pressure of the system above the solvent's vapour pressure

In one embodiment of the present invention, the contact of the non-solvent with at least a part of the first surface region film can be done by condensation of a vapour phase of the non-solvent onto a part of the first surface region film. This can be done by lowering the temperature of the vapour or by increasing the pressure of the system above the solvent's vapour pressure

In one advantageous embodiment, the solvent and/or the non-solvent comprise at least 1 dissolved substance with low vapour pressure that can be removed from the solidified surface by sublimation. Camphor and related terpenoids are examples thereof. This is advantageous for generating surface modification morphologies that are unachievable with only phase separation processes.
The concentration of said sublimatable substance in the provided solvent is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%.
The concentration of said sublimatable substance in the provided non-solvent is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%.

In one advantageous embodiment, the solvent and/or the non-solvent comprise at least 1 dissolved substance with high solubility that can be removed from the solidified surface by leaching. PEG, PVA, mono- and disaccharides are examples thereof. This is advantageous for generating surface modification morphologies that are unachievable with only phase separation processes.
The concentration of said leachable substance in the provided solvent is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%. The concentration of said leachable substance in the provided non-solvent is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%.

In one embodiment, the non-solvent comprises a dissolved second polymer that can be precipitated by contact with a second non-solvent for the second polymer.

In yet a further embodiment said second non-solvent comprises the first dissolved polymer (from step b)) and/or a third dissolved polymer. In these embodiments, a layered buildup of multiple different polymers with decreasing or alternating solvent resistance can be achieved. This is advantageous for augmenting biocompatibility, cell adhesion, drug delivery capabilities and tuning degradation rates. This embodiment is further advantageous for incorporation of bioactive agents that are labile in the solvent.

In a highly advantageous embodiment, the non-solvent is viscous and weak, e.g. glycerol dioxane 70%:30%, and is introduced to the surface film in a directed flow with a controlled speed. The viscous drag on the solidifying surface film will under certain conditions pull filamentous curtains of polymer from the underlying solid surface in the direction of the non-solvent flow. The thickness of these filaments range 3 µm to 300 µm. This is beneficial for generating additional cell attachment surface area inside scaffolds and for maintaining porous interconnectivity in at least one direction.

In a resounding advantageous embodiment, the solvent and/or the non-solvent are agitated when contacting the surface region. The forms of usable agitation include stirring, shaking, perfusion, sonication. Agitation is advantageous because it both accelerates solvent non-solvent exchange and mechanically shapes the solidifying surface region film.

In yet another embodiment, the solidified modified surface can be sonicated to further mechanically alter the porous surface. This is advantageous for generating beneficial surface modification morphologies that are unachievable if using only phase separation processes.

In another advantageous embodiment, the non-solvents comprise a dissolved polymer and at least one bioactive agent.

In an additional advantageous embodiment, the outermost layer formed through said multilayered process comprises one or more agents that inhibit surrounding cell growth such as chemotherapeutic agent such as doxorubicin, cisplantin, paclitaxel. The underlying layer can contain an agent that enhance cell growth either directly in the case of specific growth factors including but not limited to BMP-2, FGF, SCF, or SDF-1 or indirectly by promoting a more cell-friendly local environment by promoting vascularization using among other things VEGF or chemotactic agents. The invention in this embodiment is advantageous for treatment and prevention of tumor recurrence following resection, where the bioactive agent in the first layer will eliminate remaining malignant cells and the the second layer will promote tissue regeneration.

In yet an additional embodiment, the outermost layer comprises an antibiotic for prevention of infection at the site of device.

In the case where the unmodified medical device is a macro-porous scaffold, such as a tissue scaffold, the purging step (the non-solvent step) of the present invention especially takes advantage of the highly interconnected, CAD-optimized geometry of rapid prototyping scaffolds and allows for preservation of the open pore structure of the unmodified scaffold. In many cases the shape of the medical device has been optimized using finite element analysis and/or computational fluid dynamics analysis.

The unmodified scaffold can be conventionally fabricated using a variety of rapid prototyping i.e. additive manufacturing methods including fused deposition modelling (FDM), selective laser sintering, stereolithography, and 3D printing. Rapid prototyping methods, especially those utilizing thermal processing, usually produce mechanically stable constructs with limited geometry detail; an increase in detail generally increases production time.
Principally, the base scaffold can be fabricated using many other methods including but not limited to thermally induced phase separation with lyophilization, spinning, weaving, etching, felting, knitting, injection molding, blow molding, machining, micromaching, laser micromachining, fiber extrusion, particulate leaching, lost mould casting, and bead sintering. In the present context, the term "base scaffold" is to be understood as the unmodified scaffold that has not yet been processed by the method of the present invention.

Polymeric RP scaffolds are available commercially. A non-exhaustive list includes: 3D Biotek LLC (NJ, USA); Reinnervate Limited (UK); Osteopore International (Singapore) (PCL+tricalcium phosphate scaffold has been used clinically); CELLTREAT Scientific Products LLC (MA, USA). Such scaffolds are suitable for modification according to the present invention.

The fundamental requirement of the unmodified medical device for it to be usable in the process of the present invention is that its surface region comprises one or more types of polymers that have a solvent-non-solvent pair.
Preferably, the solvent and non-solvent are miscible at temperatures below the melting point of the polymer present in the surface region. A very large number of materials fulfill such a condition. Non-limiting examples of such polymers that are also biocompatible are poly(ester amide), polyhydroxyalkanoates (PHA), poly(3-hydroxyalkanoates) such as poly(3- hydroxypropanoate), poly(3-hydroxybutyrate), poly(3-hydroxyvalerate), poly(3- hydroxyhexanoate), poly(3-hydroxyheptanoate) and poly(3-hydroxyoctanoate), poly(4- hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, poly(D,L-lactide), poly(L-lactide), polyglycolide, poly(D,L- lactide-co-glycolide), poly(L-lactide-co-glycolide), polycaprolactone, poly(lactide-co- caprolactone), poly(glycolide-co-caprolactone), poly(dioxanone), poly(ortho esters), poly(anhydrides), poly(tyrosine carbonates) and derivatives thereof, poly(tyrosine ester) and derivatives thereof, poly(imino carbonates), poly(glycolic acid-co-trimethylene carbonate), polyphosphoester, polyphosphoester urethane, poly(amino acids), polycyanoacrylates, poly(trimethylene carbonate), poly(iminocarbonate), polyphosphazenes, silicones, polyesters, polyolefins, polyisobutylene and ethylene- alpha-olefin copolymers, acrylic polymers and copolymers, vinyl halide polymers and copolymers, such as polyvinyl chloride, polyvinyl ethers, such as polyvinyl methyl ether, polyvinylidene halides, such as polyvinylidene chloride, polyacrylonitrile, polyvinyl ketones, polyvinyl aromatics, such as polystyrene, polyvinyl esters, such as polyvinyl acetate, copolymers of vinyl monomers with each other and olefins, such as ethylene-methyl methacrylate copolymers, acrylonitrile-styrene copolymers, ABS resins, and ethylene-vinyl acetate copolymers, polyamides, such as Nylon 66 and polycaprolactam, alkyd resins, polycarbonates, polyoxymethylenes, polyimides, polyethers, poly(glyceryl sebacate), poly(propylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(m ethyl methacrylate), epoxy resins, polyurethanes, rayon, rayon-triacetate, cellulose acetate, cellulose butyrate, cellulose acetate butyrate, cellophane, cellulose nitrate, cellulose propionate, cellulose ethers, carboxymethyl cellulose, polyethers such as poly(ethylene glycol) (PEG), copoly(ether-esters) (e.g. poly(ethylene oxide-co-lactic acid) (PEO/PLA)), polyalkylene oxides such as poly(ethylene oxide), poly(propylene oxide), poly(ether ester), polyalkylene oxalates, phosphoryl choline, choline, poly(aspirin), polymers and copolymers of hydroxyl bearing monomers such as 2-hydroxyethyl methacrylate (HEM A), hydroxypropyl methacrylate (HPMA), hydroxypropylmethacrylamide, PEG acrylate (PEGA), PEG methacrylate, 2-methacryloyloxyethylphosphorylcholine (MPC) and n-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene- PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), Pluronic(TM) surfactants (polypropylene oxide-co-polyethylene glycol), poly(tetramethylene glycol), hydroxy functional poly(vinyl pyrrolidone), biomolecules such as collagens, silk fibroin, chitin, chitosan, alginate, fibrin, fibrinogen, cellulose and cellulose derivatives, starch, dextran, dextrin, hyaluronic acid, fragments and derivatives of hyaluronic acid, heparin, fragments and derivatives of heparin, glycosamino glycan (GAG), GAG derivatives, polysaccharide, elastin, or combinations thereof. In some embodiments, the coatings can exclude any one of the aforementioned polymers.

In an embodiment the surface region of the medical device is non-metallic.
As used herein, the terms poly(D5L-lactide), poly(L-lactide), poly(D,L-lactide-co-glycolide), and poly(L-lactide-co-glycolide) can be used interchangeably with the terms poly(D,L-lactic acid), poly(L-lactic acid), poly(D,L-lactic acid-co-glycolic acid), or poly(L-lactic acid-co-glycolic acid), respectively.

In one embodiment, the polymer in selected from the group consisting of poly(lactic acid), poly(lactic acid -co-glycolic acid) (PLGA),
poly(glycolic acid), poly(lactic acid-co-caprolactone) (PLCL), poly(E-caprolactone) (PCL), poly(butyric acid), poly(butyric acid-co-valeric acid) and mixtures thereof.

In one embodiment of the present invention, the solvent and non-solvent of the biocompatible polymer are miscible at temperatures below the melting point of the biocompatible polymeric composition.

In another embodiment of the present invention, the surface region comprises calcium phosphate and/or calcium carbonate, such as TCP (Tri-Calcium Phosphate) and HA (hydroxyapatite).

In yet another embodiment of the present invention, the solvent of the biocompatible polymer comprises a bioactive agent for incorporation into the surface region.

The modified surface morphology (e.g. augmented porosity) makes the solidified surface region film much more susceptible to hydrolytic treatments with e.g. sodium hydroxide solution. Preliminary results show that sodium hydroxide treatments can tune the stiffness of the solidified surface region film without altering the global strength of the medical device (in this case, a tissue scaffold) or causing overt changes to the computer-geometry of the medical device.

Hence, in one embodiment of the present invention, the method comprises the step of:
- subjecting the solidified surface region film to a hydrolytic treatment.
In another embodiment, the hydrolytic treatment is performed with a basic solution. In the present context, the term 'basic solution' refers to a solution comprising a base.

In another embodiment, the hydrolytic treatment is performed with an aqueous basic solution. In the present context, the term 'aqueous basic solution' refers to an aqueous solution containing more OH⁻ ions than H⁺ ions, i.e. an aqueous solution with a pH greater than 7. In one embodiment of the present invention the pH of the basic solution is within the range of 8-14, e.g. 9-13, such as within the range of 10-12.

Preferably, the aqueous basic solution comprises a strong metal hydroxide, such as an alkali metal or alkaline metal hydroxide, including, but not limited to Group I or II metals such as sodium hydroxide, potassium hydroxide, lithium hydroxide, calcium hydroxide, strontium hydroxide, magnesium hydroxide, and the like.
The inventors have found that the ability to tune the stiffness and surface morphology of a tissue scaffold, and thereby stem cell differentiation, contributes to developing the desired tissue faster, thereby shortening the time a cell seeded tissue scaffold (implant) needs to be cultured before use, or if implanted immediately, presumably the time before a defect is healed.

The surface modified medical device has a highly increased surface area compared to the unmodified medical device. BET surface area analysis have been conducted on tissue scaffolds with a height of 5 mm and a diameter of 10 mm (see examples section): The tissue scaffolds have a measured surface area of approximately 60,000 mm²/scaffold, while the unmodified scaffolds have a calculated surface area of approximately 2,000 mm²/scaffold.
Consequently, a surface modified medical device according to the present invention must have a higher capacity for sorption or conjugation of a variety of bioactive agents (e.g. therapeutic molecules and drug carriers), the addition of which will turn the scaffold into a localized drug delivery system. These substances include, but are not limited to:
1) Non-encapsulated, non-conjugated agents for e.g. chemotherapy or antibiotic treatment;
2) Signalling molecules for local control of cell and tissue response;
3) Components of extracellular matrix to mimic the native environment of adherent cells;
4) Viral vectors for gene therapy;
5) Polymeric drug carriers;
6) Micelle drug carriers;
7) Vesicle drug carriers;
8) Nano- and microparticle carriers.

In one embodiment of the present invention, the method further comprises the step of:
- sorbing a bioactive agent onto the solidified surface region film.

This may be done both with and without the hydrolytic treatment. Thus in an embodiment the one or more bioactive agents are added to the device prior or post hydrolytic treatment by means of physical adsorption or by specific binding to agents incorporated in the surface.

In an embodiment the medical device obtainable by the process according to the invention has an overall shape which is incompatible with molding, casting, plating, embossing, blow molding, or subtractive manufacturing. E.g. porous walled hollow sphere comprises pores with defined size distribution suspended by struts inside a larger hollow sphere with a different size of pores.

In another embodiment of the present invention, the bioactive agent is selected from the group consisting of antiviral agents, antimicrobial agents, angiogenic agents, antigenic agents, antitumor agents, immunosuppressants, vaccines, vaccine adjuvants, psychotropic drugs, anti-depressants, anti-psychotics, tranquilizers, analgesics, anaesthetics, stimulants, depressants, mood stabilizers, anxiolytics, sex hormones, caffeine, nicotine, opioids, barbiturates, benzodiazepines, growth factors, amino acids, nucleic acids, vitamins, hyaluronic acid, bone morphogenic proteins, hormones, enzymes, bone autograft, analgesics, bone marrow, bone allograft, hydroxyapatite, parenchymal cells, and mesenchymal cells or combinations thereof.

In still another embodiment of the present invention, the bioactive agent is selected from the group consisting of antiviral agents, antimicrobial agents, angiogenic agents, antigenic agents, antitumor agents, immunosuppressants, proteins, peptides, steroids, viral vectors, growth factors, amino acids, polyribonucleic acids, polydeoxyribonucleic acids, chemically modified polynucleic acids, vitamins, hyaluronic acid, bioactive carbohydrates, bioactive lipids bone morphogenic proteins, hormones, enzymes, bone autograft, analgesics, bone marrow, bone allograft, hydroxyapatite, calcium phosphate, calcium carbonate particles, tricalciumphosphate, bioglass, strontium compounds, parenchymal cells, and mesenchymal cells or combinations thereof.

In still another embodiment of the present invention, the bioactive agent is selected from the group consisting of a second polymer (different from the polymer present in the medical device); nano- or microparticulates of a second or more undissolved polymers;synthetic oligomers; cross-linking agents; antibodies; chelators; clays; silicates; bioresorbable glass particles; dyes; fluorescent dyes; surfactants; detergents; wetting agents; emulsifiers; foaming agents; and dispersants; flocculating agents; salts; buffering agents; quantum dots; Radiotherapeutic Nuclides; and radiopacifiers.

In still another embodiment of the present invention, the bioactive agent is combined with delivery vehicles, targeting agents, endosomal escape agents, subcellular targeting agents, controlled release vehicles, or mixtures thereof.

In the present context, the term 'delivery vehicle' refers to any vehicle that is capable of protecting and stabilizing the bioactive agent against molecules, cells or environments that act detrimental to the agent or its delivery to the site of action while delivering the bioactive agent past biological barriers such as but not limited to the cell membrane, blood brain barrier, endothelial wall, gut wall, kidney, liver, spleen or the reticuloendothelial system.

In the present context, the term 'targeting agent' refers to any agent that will promote the bioactive agent to reach and/or interact with its target organs and/or cells. Examples include aptamers, antibodies and natural receptor ligands.

In the present context, the term 'endosomal escape agent' refers to any agent that will allow the bioactive agent to escape from the endosome.

In the present context, the term 'subcellular targeting agents' refers to any agent that will promote the delivery of the bioactive agent to desired sub cellular compartment such as the cytoplasm, nucleus or exosomes. Examples include signalling peptides, snare proteins and nuclear localization signals.

In the present context, the term 'controlled release vehicles' refers to any agent that will release another agent in a delayed or prolonged fashion.

In another embodiment of the present invention, the bioactive agent is added to the solvent of the biocompatible polymer prior to use. This allows for incorporation of the bioactive agent into the solidified surface region film. Hence, in one embodiment, the solvent of the biocompatible polymer comprises a bioactive agent.

In still another embodiment of the present invention, the bioactive agent is added to the non-solvent of the biocompatible polymer prior to use. Hence, in one embodiment, the non-solvent of the biocompatible polymer comprises a bioactive agent.

In still another embodiment of the present invention, the solvent and/or non-solvent of the biocompatible polymer comprise a bioactive agent.

In one embodiment of the present invention, the solvent of the biocompatible polymer comprises calcium phosphate and/or calcium carbonate particles.

In another embodiment of the present invention, the bulk part of the medical device comprises a biocompatible polymeric composition identical to the biocompatible polymeric composition in the surface region.

In one embodiment of the present invention, the medical device is one or more polymeric particles. Implantable, ingestible, inhalable or injectable particles with increased surface area may act for examples as drug carriers or depots for sustained drug release. This may increase patient compliance and satisfaction.

The thickness of the solidified surface region film can also be controlled by tuning the temperature of the solvent and the contact time between the solvent and the surface region of the unmodified medical device. Therefore, in still another embodiment according to the invention, the depth/thickness of the solidified surface region film is a function of contact time and temperature. Hence, the thickness can be tuned according to specific requirements.

The inventors of the present invention have completed experiments where a buffer solution containing various nanoparticles or proteins was adsorbed onto a scaffold modified by the process of the present invention, and lyophilized before studying release kinetics in vitro.
One PCL based embodiment of the scaffold has demonstrated vastly improved retention of a range of nanoparticles and proteins which encourages its use in regenerative medicine where the combined use of scaffolds and drugs is necessary for optimal function and integration of an implant. Since scaffolds prepared according to the present invention are able to retain drugs that can be loaded gently onto the scaffolds, they are well-suited for locally delivering drugs that are too sensitive for conventional methods for incorporating drugs into polymeric delivery matrices. Furthermore, the ease of drug loading also enables individually tailored drug functionalization of scaffolds in the clinic. The improved drug retention demonstrated by the scaffolds prepared according to the present invention means that less drug needs to be included with the implant to achieve the desired effect. This is important as the drugs used are often very expensive and have risk of side effects if they rapidly leak from the implant into the surrounding tissue.

A medical device prepared according to the present invention makes better use of the expensive cells and drugs that are being employed in regenerative medicine thereby lowering costs, improving safety and shortening the delay before a sick patient can be treated.

Hence, one aspect of the present invention relates to a medical device obtainable by the method of the present invention.

In one embodiment of the present invention, the surface region of the medical device has a rough or coarse morphology or a morphology with channels or pores.

In one embodiment the device is a 3D cell culture substrate for stem cell culture, tissue engineering, drug discovery, drug testing, high throughput drug screening, bioproduction, anti-body production, protein production, and broad cell biology applications.

In an embodiment the device is for bioreactor bioproduction for filtration and adsorption of proteins, sugars, or other secreted cell products.

In the field of orthopedics, it is often desirable to fill bony defects or voids and to deliver therapeutic agents to such sites. Such defects can be the direct result of disease or trauma, removal of diseased tissue or tumors, osteolytic conditions caused by wear debris from a prosthetic joint, or other degenerative or damaging conditions. Common sites that can present void defects include the cranium, fracture sites (especially compound fracture sites), areas comprising and proximate to synovial joints, and attachment sites for prosthetic joints.

Bone graft is used to fill spaces in bone tissue that are the result of trauma, disease degeneration or other loss of tissue. Clinicians perform bone graft procedures for a variety of reasons, often to fill a bone void created by a loss of bone or compaction of cancellous bone. In many instances, the clinician must rely on the bone graft material to provide some mechanical support, as in the case of subchondral bone replacement or compaction grafting around total joint replacement devices. In these instances, clinicians pack the material into the defect to create a stable platform to support the surrounding tissue and hardware.

Thus, in an embodiment the medical device is a bone tissue engineering scaffold. In yet an embodiment the medical device is biodegradable.

There are several options available to the orthopaedic clinician for bone graft material. Most commonly, the source of the graft material is either the patient (autograft), which is clinically preferable, or a donor (allograft). However, autograft has the potential drawback of increased pain and morbidity associated with a second surgical procedure, in addition to having a limited supply of the bone. In autograft and, to a lesser extent, in allograft there are biological factors such as proteins or cells that are present that can assist in the fracture healing process. Xenografts and bone graft substitutes are other options.

In one embodiment of the present invention, the medical device is arranged for a bone-graft substitute composition; wherein the medical device has a granule size within the range of 1-10 mm.

A synthetically derived substitute material has advantages over human derived bone graft and naturally derived substitutes, including: 1) more control over product consistency; 2) less risk for infection and disease; 3) no morbidity or pain caused by harvesting of the patient's own bone for graft; and 4) availability of the substitute in many different volumes (that is, it is not limited by harvest site of the patient).

The bone graft substitute materials that have been used commercially exhibit various levels of bioactivity and various rates of dissolution. Bone graft substitute products are currently available in several forms: powder, gel, slurry/putty, tablet, chips, morsels, and pellets, in addition to shaped products (sticks, sheets, and blocks). In many instances, the form of bone graft substitute products is dictated by the material from which they are made. Synthetic materials (such as calcium sulfates or calcium phosphates) have been processed into several shapes (tablets, beads, pellets, sticks, sheets, and blocks) and may contain additives such as antibiotics (e.g., Osteoset(R)-T (Wright Medical Technology; Arlington, Tenn.)) or bioactive agents (e.g., Rhakoss(R) (Orthovita(R); Malvern, Pa.)). Allograft products, in which the source of the bone graft material is a donor, are typically available as chips and can be mixed with a gel to form a composite gel or putty.

In another embodiment, the bone-graft substitute composition is in the form of a gel or putty. The term 'putty' is to be understood as a substance having a uniform consistency that is solid but moldable and pliable. The putty of the instant invention may be molded and shaped. Preferably, the putty is capable of remembering its shape.

In other embodiments, the bone-graft substitute composition is in the form of a tablet, a bead, a pellet, a stick, a sheet, or a block.

In still another embodiment of the present invention, the solidified surface region film has a depth of at least 2 micrometres.

Polymeric scaffolds are intended to function as a temporary extracellular matrix (ECM) until regeneration of bone or other tissues has occurred. Therefore, the more closely it resembles an in vivo microenvironment, the more likely the success of the scaffold.

State-of-the-art polymeric scaffolding has been considered for the formation of 3D implant models that fit into specific defects. This is mainly obtained by fused deposition methods copying a 3D scanning image. Materials used are pure polymers, mixed polymers, or mixture of polymer and e.g. bone-conducting granules. But the precision deposition is restricted by the limited surface area available for cell attachments onto individual fibres.

Hence, in the present invention, a combination of the strict 3D structure with an interior providing abundant surface area for cell ingrowth is proposed as a new and reasonable solution. The abundant surface area is obtained by applying the process of the present invention onto a polymeric scaffold.

In another embodiment of the present invention, the medical device comprises a biocompatible material shaped as a framework forming one or more open networks of voids, such as a 3D printed tissue scaffold.

In yet another embodiment of the present invention, the medical device is selected from the group consisting of bone scaffold, stent, self-expandable stent, balloon expandable stent, stent-graft, graft, aortic graft, heart valve prostheses, cerebrospinal fluid shunt, pacemaker electrode, catheter, endocardial lead, anastomotic device, CABG anastomotic clips and connectors, orthopedic implant, screw, spinal implant, and electro-stimulatory device.

Even though the technology of the present invention is especially useful within the field of medical devices, this technology may also be useful for other applications.

Hence, one aspect of the present disclosure describes a method for modifying a surface morphology of an article comprising:
a) providing an article comprising a bulk part and a surface region, said surface region comprises a biocompatible polymeric composition comprising a biocompatible polymer;
b) contacting at least a portion of the biocompatible polymeric composition in said surface region with a solvent of the biocompatible polymer to form a first surface region film on at least a first part of said surface region;
c) contacting at least a part of said first surface region film with a non-solvent of said biocompatible polymer, thereby forming a first solidified surface region film on at least a part of said surface region, with the proviso that said article not being a medical device.

Another aspect of the present invention relates to an article obtainable by the method of the resent invention.

More claims pertaining to increased anchoring stability for a second component due to increased porosity.

In one embodiment of the present invention, the at least a part of the surface region comprising the first solidified surface region film is coated with a second component.

In another embodiment, the second component is selected from the group consisting of a synthetic or biological monomer solution, synthetic or biological polymer solution, gel, hydrogel, hydrocolloid, polyelectrolyte solution, emulsions, colloidal suspension, oligomers comprising oligo pnipaam and combinations thereof.

The second component may be deposited superficially and/or profoundly onto/into the surface region.

In another embodiment, the second component does not dissolve the first solidified surface region film.

In one embodiment, the second component forms a coating on the internal surfaces maintaining pore interconnectivity. Preferably, said second component must not obliterate macroscopic pore space, i.e. maintains pore interconnectivity.

The second component may be thermally induced to phase separate and lyophilize after being brought into contact with the surface region of the surface treated medical device. Alternatively, it may be introduced by the already disclosed process of the present invention.

It should be noted that embodiments and features described in the context of one of the aspects of the present invention also apply to the other aspects of the invention.

The invention will now be described in further details in the following non-limiting examples.

### Examples

### Example 1

### Scaffold fabrication

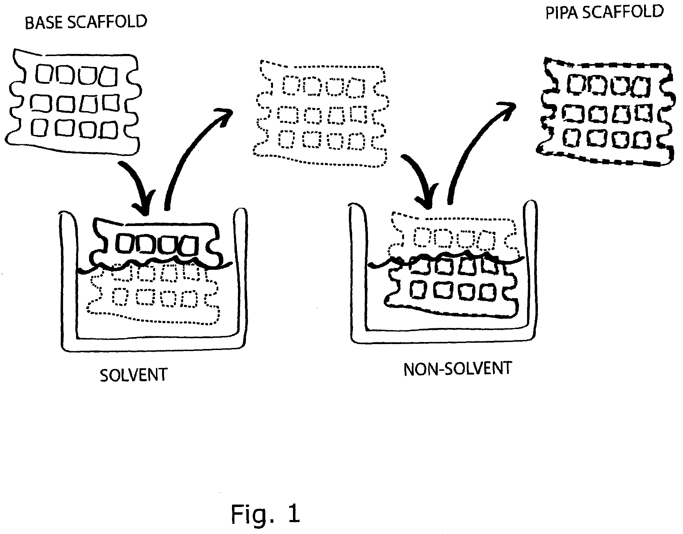

Fused deposition modelling (FDM) scaffolds were made from 50 kDa polycaprolactone (Perstorp, UK) using a BioScaffolder (sys-eng GmbH, Germany). 2 mm high mats were comprised of approximately 170 µm thick fibers with a center-to-center spacing set to 1.00 mm. Individual scaffolds were punched out of the mats using a 4 mm biopsy punch (Miltex, Japan).
The scaffolds were arranged in a stainless steel mesh basket and immersed in p-dioxane (1,4-Dioxane, Sigma-Aldrich, Denmark) for 10 seconds and then quickly immersed in a coagulation bath comprising a large volume of 70 % ethanol (the non-solvent) under gentle stirring (Figure 1). The non-solvent was replaced two times for each 30 minutes with a subsequent bath in sterile water for 30 minutes. For the following 3 hour hydrolytic treatment with 5 M sodium hydroxide, the precipitated scaffolds were gently blown with N₂ to remove excess water between the scaffold fibers. Care was taken to avoid drying out the scaffolds completely as this would impede wetting by the sodium hydroxide solution. Hydrolysis was ended by multiple baths in PBS (buffer with neutral pH) until the pH had stabilized at 7.4. The scaffolds again blown with N₂ to remove excess PBS and washed in sterile water avoid deposition of NaCl on the surface. A possible setup can be seen in Figure 1.

### Experimental parameter sweep

A fabrication parameter sweep was conducted for the variables: solvent composition (dioxane/EtOH ratio v/v), solvent exposure time, and coagulation bath composition i.e. non-solvent mixture (EtOH/water v/v ratio) (Table 2). The sweep was conducted at room temperature (20°C) and explored the morphology of the combinations as set forth in Table 2 (solvent, time, coagulation).

**Table 2: Combinations of processing parameters**

| | | | | | |
|---|---|---|---|---|---|
| Solvent | 60/40, 10 s, 70/30 | 70/30, 10 s, 70/30 | 80/20, 10 s, 70/30 | 90/10, 10 s, 70/30 | 100/0, 10 s, 70/30 |
| Time | 100/0, 5 s, 70/30 | 100/0, 10 s, 70/30 | 100/0, 20 s, 70/30 | 100/0, 40 s, 70/30 | 100/0, 80 s, 70/30 |
| Coagulation | 100/0, 10 s, 0/100 | 100/0, 10 s, 30/70 | 100/0, 10 s, 50/50 | 100/0, 10 s, 70/30 | 100/0, 10 s, 100/0 |

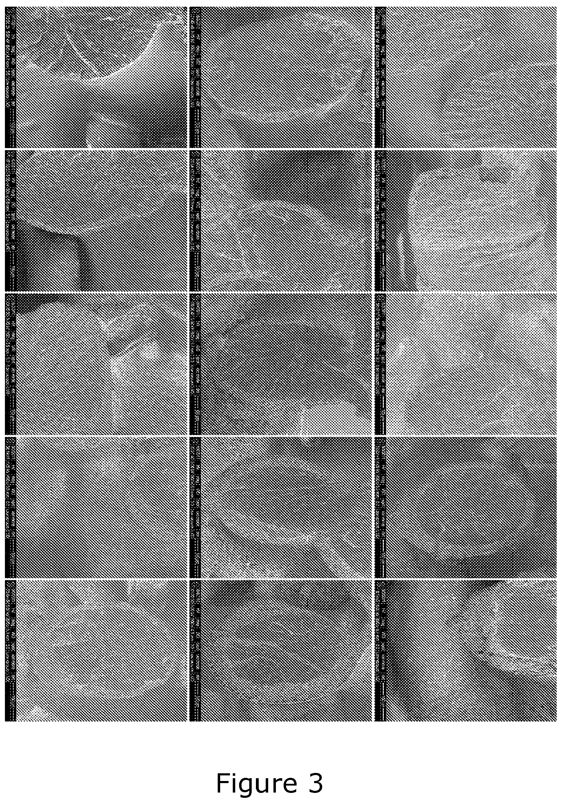

Four scaffolds were taken from each combination for scanning electron microscopy (Novasem_600, FEI, OR). Each scaffold was immersed in liquid nitrogen to induce glass transition and immediately cleaved to provide a clean fracture of the scaffold and porous coating. Representative images are arranged in Fig 3.

The effects of further lowering the non-solvent power of the coagulation bath were probed with a single combination of 100/0 dioxane solvent, 10 seconds exposure, and a coagulation bath consisting of dioxane-EtOH mix with a ratio of 60/40 (Fig.5)
Isolated experiments to investigate the effects of coagulation bath temperature were also conducted (Fig. 5).
The method was tested on scaffolds with smaller pores to see if interconnectivity and scaffold architecture could be preserved despite thinner fibers and higher fluid resistance to the flowing non-solvent.

### BET surface area analysis

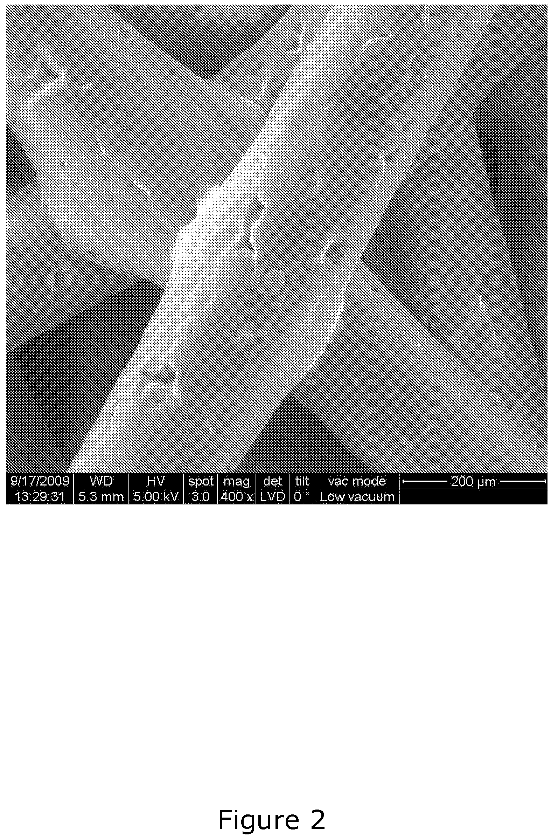

The scaffolds treated by the method of the present invention were degassed for 48 hrs. at 40°C under vacuum. BET surface area analysis was performed with nitrogen (NOVA-e 2200, Quantachrome, FL). The specific surface area was calculated from the shape of the sigmoidal adsorption curve and the mass of the measured scaffolds. Similar analysis on control PCL scaffolds was not possible due to surface area below detection threshold and the specific surface area was calculated from SEM and micro-CT data. The treatment produced a specific surface area of 0.449 m²/g, which equals a 30-fold increase compared to the control scaffold (Figure 2).

### Protein and nanoparticle release

### Transfection reagent nanoparticle formation

Three commercially available transfection reagents oligofectamine, lipofectamine2000 and TransIT-TKO were used to formulate nanoparticles for siRNA incorporation within the scaffolds. Oligofectamine and lipofectamine2000 complexes were made by mixing 3.75 µL 20 µM Cy5 labelled siRNA with 125 µL serum free medium and 3.75 µL transfection reagent with 125 µL serum free medium in separate tubes for 5 min prior to mixing and further 20 min incubation at room temperature. TransIT-TKO particles were made by mixing 15 µL TransIT-TKO with 250 µL serum free medium for 5 min after which 3.75 µL 20 µM Cy5 labeled siRNA was added, the sample was incubated 15 min at room temperature.

### Drug loading

Twenty five microliters of bovine serum albumin or lysozyme solution (1mg/ml in ddH₂O) or transfection complexes were added to scaffold cylinders (ø=4 mm, h = 2 mm). Approximately 20 µL could be adsorbed into the scaffolds when they were flipped repeatedly in the solution. Twenty minutes after loading the scaffolds were frozen on dry ice and freeze dried.

### Release Assa y

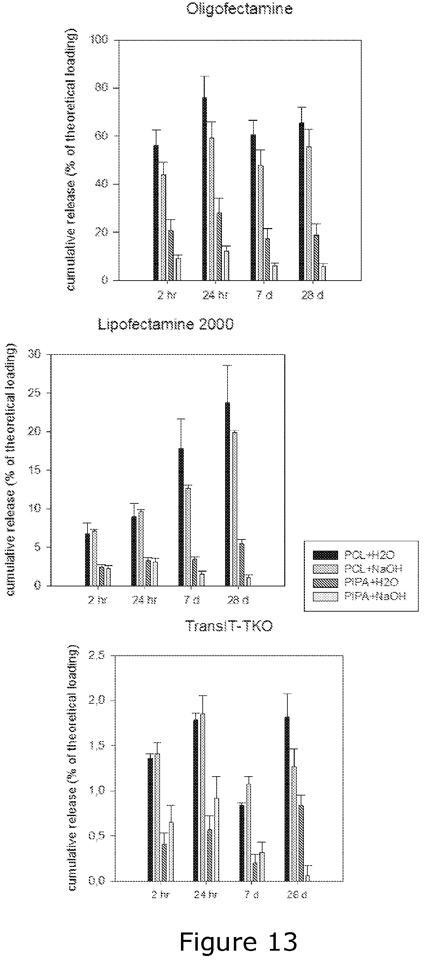

Protein or siRNA loaded scaffolds were placed in wells on a 96 well plate and were added 300 µL PBS with 0.05% NaN₃. The plates were then sealed with parafilm, covered with aluminium foil and placed at 37C. 100 µL were removed from each well at 2 hours, 24 hours and placed in new 96 well plates that were frozen to - 20C. The removed volume was replaced. Results are shown in figure 13.

After the experiment was completed each siRNA containing plate was added a serial dilution of Cy5 labeled siRNA in PBS + 0.05% NaN₃. The plates were then scanned on a Typhoon scanner for Cy5 fluorescence (settings were Sensitivity: High, Pmt: 400, resolution: 100 µm). The resulting picture was then quantified by finding the average pixel intensity in each well and comparing this value to a standard curve made from the serial dilutions.

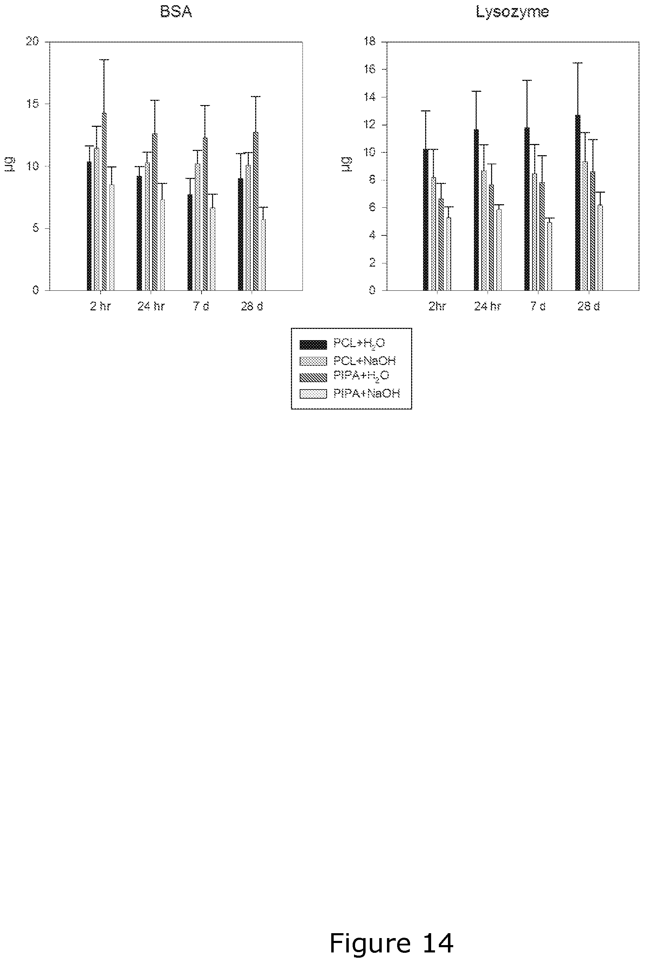

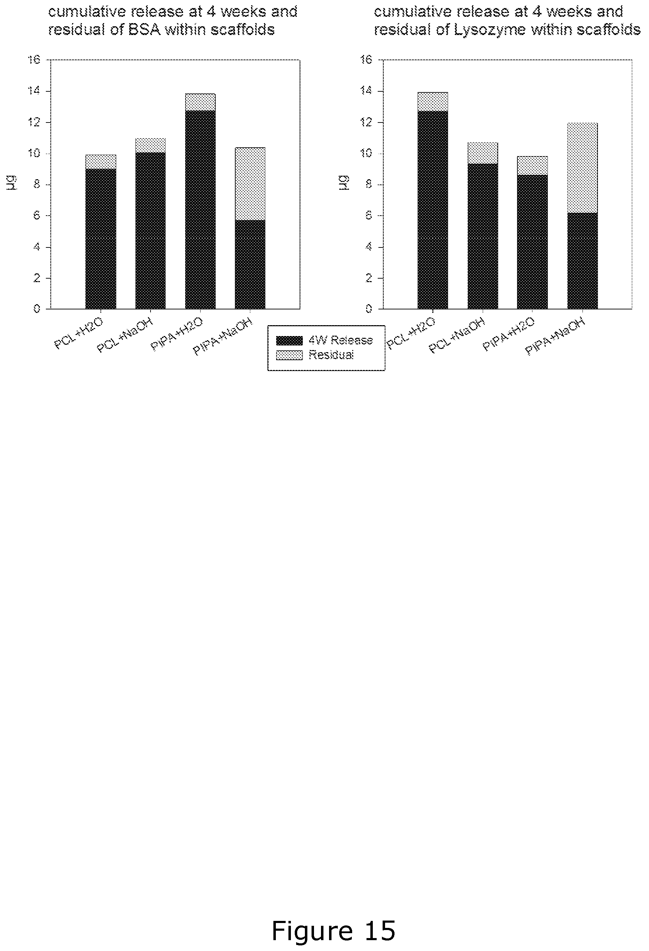

The released protein was quantified by placing 25 µL of the released material or of separate serial dilutions of each protein in wells on a 96 well plate and then adding 225 µL bradford reagent to each well. Absorbance was measured on a µQuant 96 well plate reader at 595 nm and 450nm and the 595/450 ratio was used. The results were quantified by comparing the absorbance values to the relevant standard curve. Results are shown in figure 14 and 15.

### In vitro studies on biocompatibility and osteogenic effects

### Cell culture

A telomerase reverse transcriptase gene-transduced cell population, hMSC-TERT cells, was used in this study. These cells maintain their functional characteristics of primary MSCs and have the capability to differentiate into mesodermal cell types (osteoblasts, chondrocytes, and adipocytes) with specific stimuli. Cells from PD level 262 (passage 45) were seeded at a density of 4000 cells/cm² in culture flasks in Dulbecco's modified essential medium (DMEM) containing 10% fetal bovine serum (FBS) and expanded in a humidified atmosphere of 37 °C and 5% CO2. After the cells reached PD level 271, passage 47 they were detached with 1.25% trypsin and 5 mM EDTA and seeded at a density of 1x106 cells/scaffold and cultured in DMEM/10% FBS with 10-8 M vitamin D. Penicillin (100 U/mL) and streptomycin (100 mg/L) was added to each group for the first week. Scaffolds were statically cultured on agarose floored wells for 48 h before perfusion culture to ensure cell attachment. Analyses were made at day 2 (all static), day 2+7, 2+14, and 2+21 (static and perfusion).
At day 2+14, 4 scaffolds from each group were transferred to osteogenic stimulaion media (DMEM/10%FBS with 100 nM dexamethasone, 290 µM ascorbic acid, and 5 mM β-glycerophosphate) (all from Sigma, Denmark) to stimulate calcium deposition for the remainder of the culture period. The rest of the scaffolds were kept in the original media formulation.
Cell free PCL and treated scaffolds were used as controls for ALP, DNA, RNA, and calcium quantification data.

### CellTracker staining

To assess cell viability, the cell/scaffold constructs were incubated for 30 min in DMEM containing 10 µM CellTrackerTM Green CMFDA (Invitrogen, Denmark). The staining media was then replaced with fresh DMEM/10 % FBS and incubated for another 30 min at 37°C. Non-fluorescent CMFDA is converted to brightly green fluorescent product when cytosolic esterases cleave off the acetates. The cell/scaffold constructs were then rinsed in pre-warmed PBS and fixed in 10 % formalin. Images were acquired using a laser scanning confocal microscope, 510 Meta (Zeiss Microimaging GmbH, Germany). The confocal settings (excitation, laser power, detector gain, and pinhole size) were identical for all images. The excitation wavelength is 488 nm and emission was detected through a bandpass filter with a range from 505-530 nm.

### Alkaline phosphatase (ALP) activity assay

Scaffolds were frozen in 1 mL MEM. Upon analysis, the samples were thawed, sonicated to disintegrate cells and deposited matrix, and then centrifuged. The supernatant was used in a colorimetric endpoint assay to quantify the rate of which ALP converts p-nitrophenyl phosphate (Sigma, Denmark) to the yellow p-nitrophenol. The absorbance of p-nitrophenol was measured by microspectrophotometry at dual wavelengths 405 and 600 nm in a 96-well plate using a microplate reader, (Victor3 1420 Multilabel Counter, PerkinElmer Life Sciences, Denmark). A standard curve was prepared from p-nitrophenol concentrations ranging from 0-0.2 mM. Technical duplicates were used for each sample. The remaining sample volumes were used for determining DNA amount.

### DNA quantification

To further break down cells and matrix, each DNA sample was added 3 mg collagenase and incubated in a 37°C water bath for 3 h. 1 mg proteinase K was added and the samples were incubated overnight at 45°C. The sample volume was diluted 1:10 in Tris-EDTA buffer and vortexed to release DNA from the scaffold. From each sample, 2 x 50 µL was aliquotted, prepared with Quant-iP™ PicoGreen® dsDNA assay (Invitrogen, Denmark), incubated 5 min in the dark, and measured in a 96-well plate according to manufacturer's instruction. Technical duplicates were used for each sample.

A human diploid cell contains 6.5 pg DNA and DNA quantification is a direct measure for the number of cells on the scaffolds.

### Calcium content assay

Calcium contents of cell-seeded scaffolds were quantified by colorimetric endpoint assay based on the complexation of one Ca²⁺ ion with two Arsenazo III molecules to a blue-purple product (Diagnostic Chemicals Limited, Charlottetown, PE, Canada). Briefly, the calcium deposition is dissolved in 1M acetic acid by placing in a shaker overnight. The samples were diluted 1:50 with ddH₂O and aliquots of 20 µl were transferred to a 96-well plate. Arsenazo III solution (280 µl) was added and incubated for 10 min at room temperature. A standard dilution series of calcium ranging from 0 to 50 µg/ml was prepared and Ca²⁺ concentration was quantified spectrophotometrically at 650 nm. Calcium content was expressed as micrograms of Ca²⁺ per scaffold.

### Histology

Scaffolds were fixed in 70% ethanol and embedded in Technovit^{®} 7100 (Ax-lab, Vedbæk, Denmark). 25 µm sections were made using a Sawing Microtome KDG 95 (Meprotech, Netherlands) and stained with 0.1% toluidine blue (Fluka, Denmark) at pH 7. Sections were viewed and photographed on a BX50 microscope with a Camedia C-5060 digital camera (All products from Olympus, Denmark).

### Statistical analysis

Results are presented as mean ± standard deviation (SD) for n=3 biological replicates. All data were analyzed using two-way ANOVA with and Tukey's multiple comparisons using XLSTAT (Aladdinsoft SARL, France). When significant main effects or an interaction between main effects were found, one-way ANOVA was used to test the difference within different scaffolds at the same timepoint. Differences between means were considered statistically significant when p-values <0.05.

### Quantitative analysis of cell density, ALP, and calcium

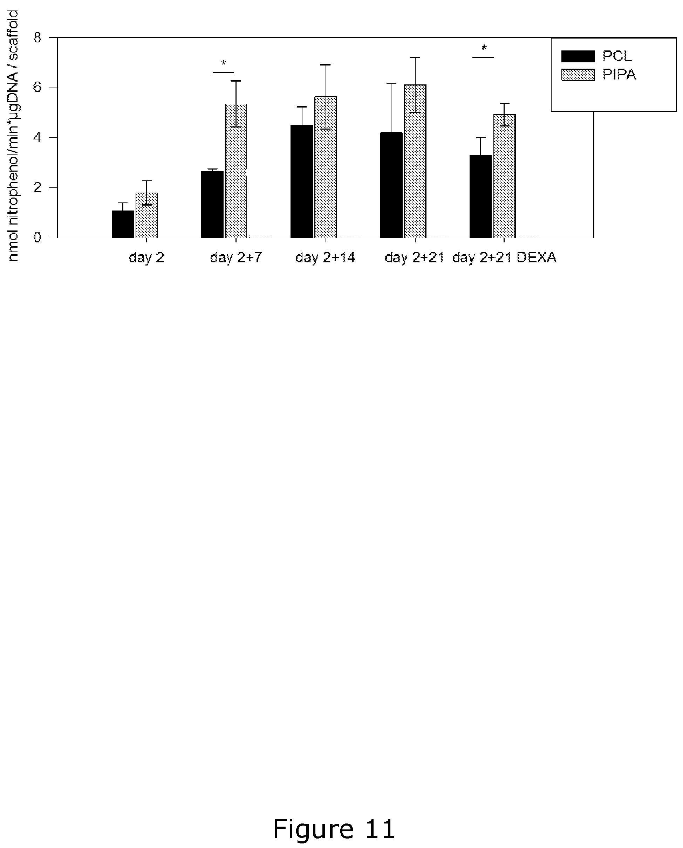

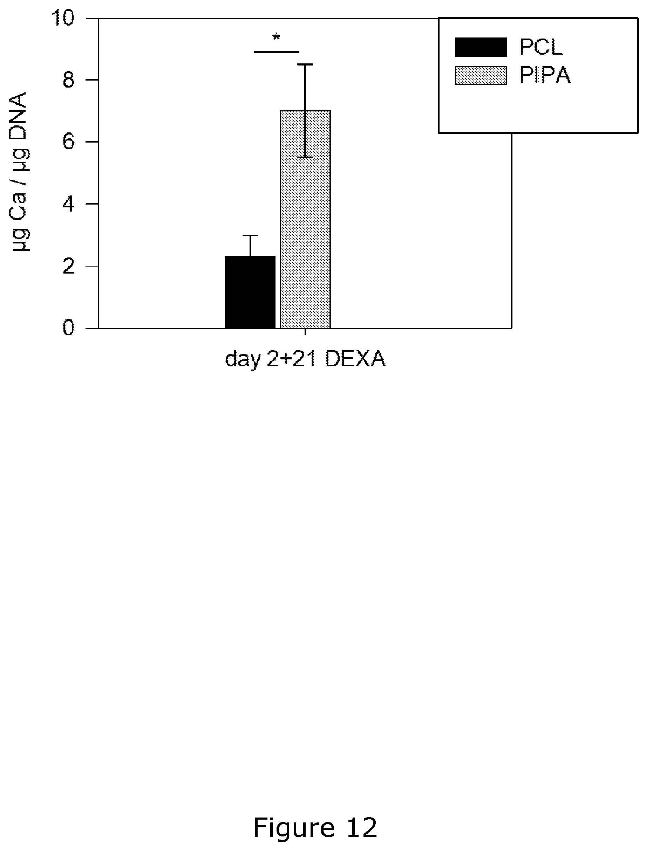

Cells proliferated faster on PCL scaffolds compared to PIPA and Perfusion. Cell number for PCL scaffolds was the highest on day 2+21 both with and without one week of DEXA stimulation. PIPA scaffolds had a slower cell growth and by day 2+21, PIPA scaffolds were comparable with Perfusion scaffolds in DEXA free media. This observation contrasts the cell abundance perceived from the confocal images and suggests a more homogeneous cell distribution in Perfusion scaffolds. Osteogenic media caused growth arrest for all three groups and cell numbers did not increase significantly from day 2+14 to 2+21 in DEXA media.
PIPA scaffolds had a remarkable early influence on ALP enzyme activity and a plateau was reached at day 2+7, Fig. 11). Surprisingly, PCL and Perfusion scaffolds were comparable. Both reached the PIPA plateau at day 2+14 and did not at any time point show any significant difference between them. DEXA media did not increase ALP activity compared to day 2+14in DEXA free media.
PIPA scaffolds had a significantly two-fold increase in calcium content compared to PCL and Perfusion scaffolds. No significant difference was found between PCL and Perfusion scaffolds (Fig. 12).

### Topographical effects on osteogenic differentiation

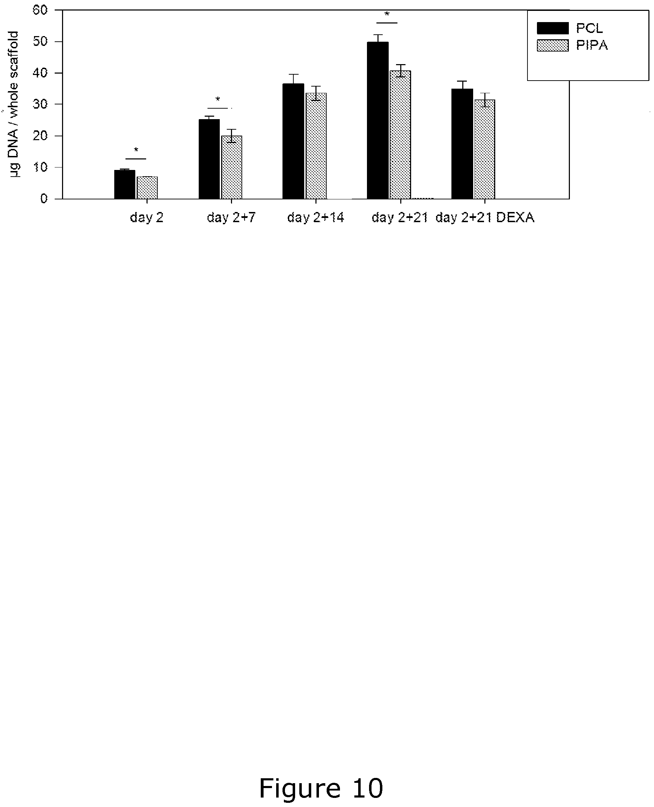

Surface roughness has long been known to modulate osteogenic responses. When plated on substrates modified with micron-sized features, cells will typically display a discernible rearrangement of focal adhesions and the actin cytoskeleton. This change is regulated by RhoA GTPase and its effector ROCK which further initiates signaling cascades that influence stem cell fate.
In this study, statically cultured treated scaffolds proved effective in enhancing osteogenic differentiation. Cell proliferation did not match that on PCL scaffolds (Fig. 10). Significantly lowered proliferation rates on day 2+7 and a corresponding peak in ALP expression on the protein level clearly indicates early onset osteogenic differentiation for a large fraction of the cells. This is possible with a substrate generated stimulus that affects all directly adherent cells. ALP expression on the transcription level was not significantly higher than PCL and suggests an induced presence of post-transcriptional regulation of the ALP gene. Calcium deposition data reflects the increased ALP activity in the treated scaffold. The data was blank-corrected to eliminate the effects of potentially increased calcium adsorption due to the change in surface structure and increased surface area.

### Example 2

### Mechanical testing

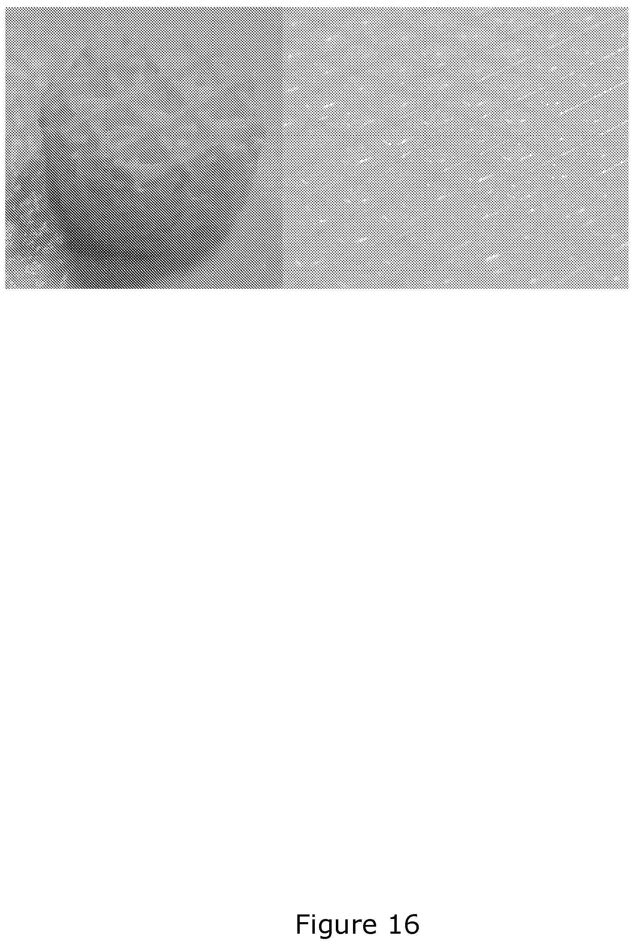

To demonstrate how medical devices according to the described invention display no discernible inferiority to prior art in terms of mechanical stiffness and strength, we made 8x8x8 mm cubic PCL scaffolds and PIPA scaffolds with identical and highly anisotropic geometry (Figure 16, right) and tested the mechanical stiffness in 3 directions with a universal testing device "Zwicki 1120" (Firm Zwick, Ulm, Germany).

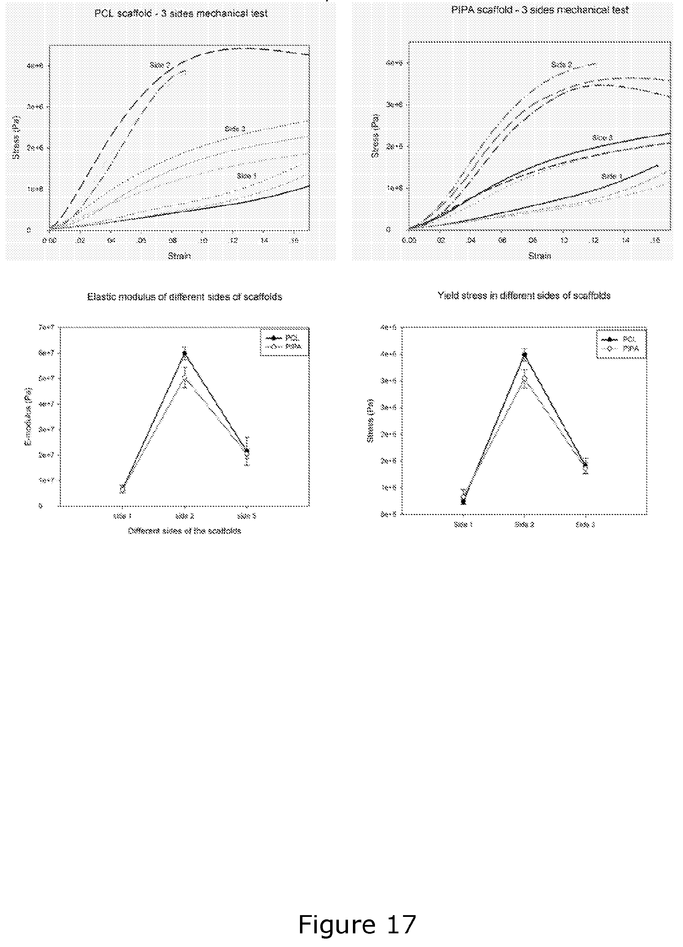

The test results show (figure 17) that the anisotropic mechanical properties of the prior art device are retained in this advantageous embodiment of the invention. Although the stiffness and yield strength for compression on side 2 is decreased, the loss can be compensated by a base scaffold with a slightly increased bulkiness.

### Example 3

### Porcine In vivo intervertebral disc application

In one advantageous embodiment, PIPA scaffolds can be used to help regenerate annulus fibrosus of the intervertebral disc (IVD). Degeneration of the IVD causes fissures and herniations in the annulus fibrosus. In order to treat the degenerated disc, the annulus fibrosus needs to heal these defects. In this study a large defect in the annulus fibrosus is sealed with 3 different scaffolds. The study is carried out in a large porcine model. The defect is applied in the antero-lateral side of 4 lumbar and 3 cervical discs. 8 mm high and 4 mm wide PIPA scaffolds are pressed into the defect and secured by sutures. After 3 months observation pigs are sacrificed, the spines harvested en bloc and frozen. Analyses include MRI, CT, histomorphology and immunohistochemistry. An implant with highly anisotropic mechanical properties such as the one mentioned previously is advantageous in this application, since the recipient tissue itself is highly anisotropic.

### Conclusion

PIPA scaffolds were implanted for 3 months during which the pigs were at full mobility and showing no signs of pain. The implant site did not show signs of

### Example 4

Doxorubicin release from two different surface morphologies To demonstrate sustained release of an active pharmaceutical ingredient

Scaffold modification: PCL scaffolds were immersed in two different 100% dioxane for 10 seconds and then quickly immersed in a non-solvent bath consisting of either 70% ethanol and 30% water (PIPA-A) or 90% ethanol and 10% dioxane (PIPA-B). Addition of a solvent of the scaffold polymer such as dioxane decreases the non-solvent power of the non-solvent bath, which ultimately facilitates a more open porous surface modification. Scaffolds were dried under vacuum to remove remaining traces of dioxane and then separated into groups with hydrolytic treatment in 5 M NaOH and without, in which case the sterile water was used instead of sodium hydroxide. Scaffolds without any treatment served as control.

### Drug loading and release:

Two different concentrations of doxorubicin were loaded into six groups of scaffolds. Drug loading efficiency was calculated. The release profiles of doxorubicin from scaffolds were monitored by incubating scaffolds in 300 µl of PBS (pH=7.4) at 37 °C for 1 month. Drug release solutions were collected and measured by a multilabel counter with ex/em at 405/615 nm.

### Cytotoxicity of drug release solution:

Osteosarcoma cells were seeded in 96-well plate (5000 cells/well) for 24 hours. Doxorubicin release solutions were added into the wells and cultured for another 24 hours. Cell viability was measured by MTT assay.

### Viability of MSCs in drug-depleted scaffolds:

MSCs were seeded into the scaffolds (4x104 cells/scaffold) after 50 days of drug release. Cell viability was tested by XTT assay at day 4, 11, and 18. Cells/scaffold constructs were fixed in 70% ethanol and stained with Hoechst staining.
Specimens were visualized under a fluorescence microscope.

### Pilot in vivo test:

Scaffolds with and without doxorubicin were implanted into dorsal muscle pockets in Danish Landrace pigs. Scaffolds were taken out after 6 weeks and used for histology study. Sections were stained with Trichrome staining.

### Results

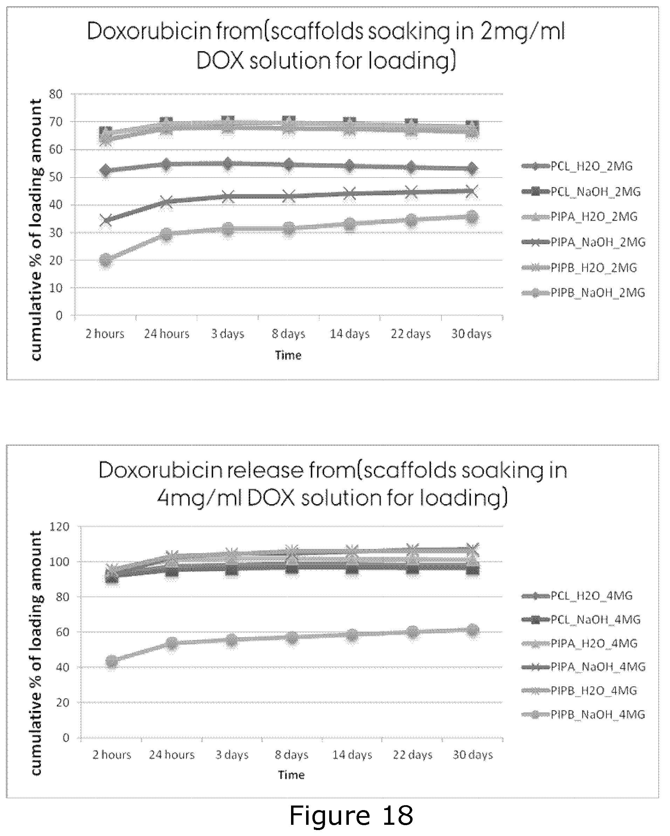

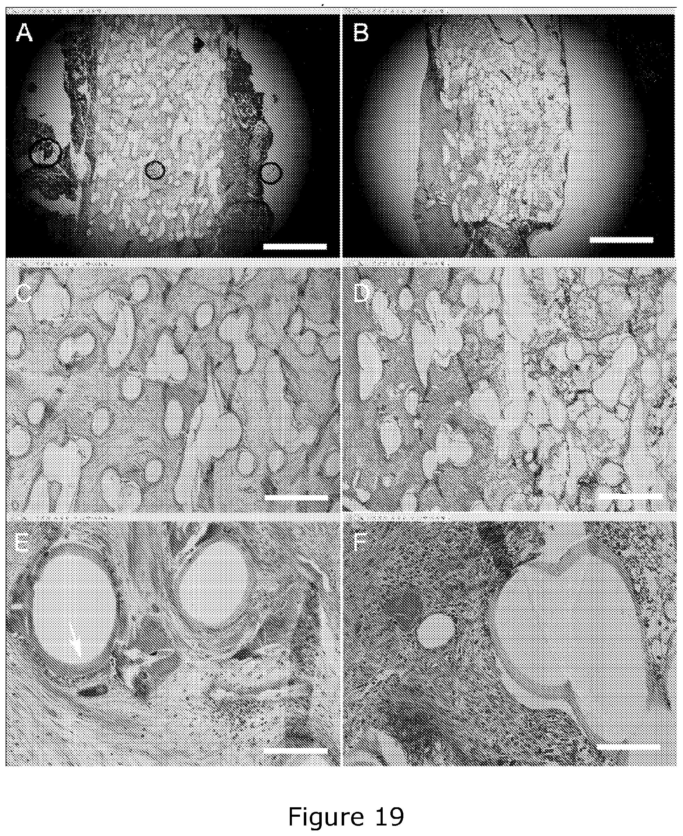

Spectrophometric measurement showed that particularly PIPA-B treated scaffolds had increased uptake of doxorubicin from the loading buffer. The enhancing effects of hydrolytic treatment on drug loading depended on the prior PIPA treatment. The following release experiment showed that hydrolytic treatment provided a stronger long term binding of doxorubicin to the scaffold. This effect was best observed from scaffolds loaded from the 2 mg/ml loading buffer. At 4 mg/ml doxorubicin loading, the release curves were dominated by the initial burst release. In both cases, PIPA-B+NaOH displayed a strongly suppressed burst release and gradual inclining release curve (Figure 18). The loaded drug loaded scaffold is locally cytotoxic throughout the implantation period as indicated by the presence of dead cells in the scaffold (Figure 19).

### Example 5

### SiRNA incorporation

In one advantageous embodiment the solvent bath or the non-solvent bath contains one or more active pharmaceutical agents that can be incorporated into the porous surface of the scaffold. The incorporation of agents will occur throughout the thickness of the precipitated layer. The release of the incorporated agents will to a lesser degree be determined by desorption and to a higher degree by the degradation kinetics of the scaffold polymer and by the microporous interconnectivity of the precipitated layer. Following example describes the incorporation of oligoethyleneimine nanoparticles containing fluorescently labeled siRNA in the surface a porous scaffold:

### Scaffold preparation:

Porous PCL scaffolds of were punched out into 8 mm wide and 4 mm high discs.

### Particle formation:

37.5uL of polymer (2.5 mg/ml OEI-HA Dioxane)
187.5 uL of Dioxane
25uL of 100uM labeled siRNA in water
Gently mix the above 3 components for 1 minute

### Scaffold coating:

Wet scaffold with particle solution for a duration of time, e.g. 10 seconds.
Immerse scaffold in non-solvent bath e.g. 100% ethanol or e.g. a mixture of 90% ethanol and 10% chloroform

Scaffolds were washed thoroughly in H₂O, and then hydrolytically treated with 2.5 M NaOH for 1h to increase hydrophilicity. Furthermore, to test the robustness of the incorporation, the scaffolds were following sonicated for 5 minutes in a glass beaker containing PBS (Bransonic 2510 ultrasonic bath). Scaffolds were then observed in an inverted fluorescent microscope.

### Results

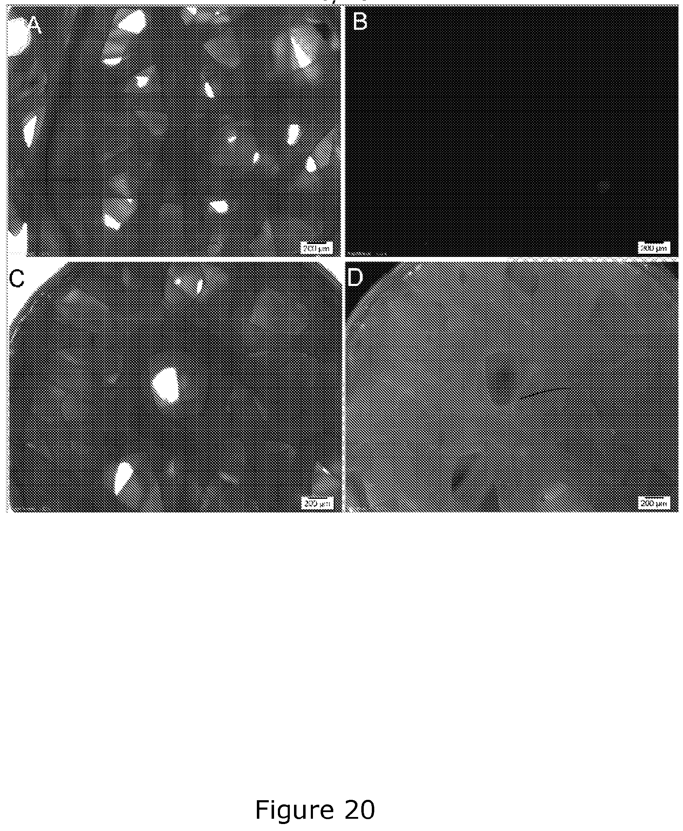

SiRNA nanoparticles were thoroughly incorporated in the scaffold by PIPA process (Figure 20). The nanoparticles remained present despite surface treatment and brief sonication.

### Example 6

PLLA fibrous nonwoven mats with a density of 65 mg/cc were punched out into 8 mm diameter and 5 mm high cylinders. The cylinders were immersed in a mixture of 50% acetone and 50% water for 2 seconds at 20 degrees Celsius then immersed in 80% ethanol 20% water bath under magnetic stirrer agitation.

SEM micrographs show that the surface of the fiber was modified with increased microporosity while the interconnectivity of the felt structure was unchanged.

### Example 7

PCL scaffolds were wetted in a mixture of 95% dioxane and 5% water for 10 seconds and then immersed in a room-temperature aqueous non-solvent bath comprising 0.1 M acetic acid and dissolved collagen for 5 minutes followed by washing in PBS.

Staining with a 5mg/mL genipin solution, which unspecifically stains lysine residues blue, indicated a presence of collagen in the modified surface region. Incorporation of matrix proteins into the surface of a medical implant is advantageous for augmenting biocompatibility.

### Example 8

PCL scaffolds were wetted in a mixture of 85% dioxane, 10% ethanol, and 5% water for 10 seconds and then immersed in a room-temperature aqueous non-solvent bath comprising 0.1 M acetic acid and dissolved chitosan with a deacetylation degree of 85% for 5 minutes followed by washing in PBS.

SEM imaging confirmed incorporation of chitosan in the surface.

### Example 9

Osteogenic differentiation of human mesenchymal stem cells on BMP2 functionalized PIPA scaffolds.

### Hypothesis

Dip coating PIPA scaffolds with BMP2 will stimulate osteogenic differentiation of human mesenchymal stem cells (hMSCs) *in vitro*

### Materials and methods

12 PIPA scaffolds (ø=4 mm, h = 2 mm) were placed in vials containing 500 µl of 10% BSA in PBS supplemented with either 20 or 100 ng/ml BMP2. After vortexing the vials were centrifuged at 200 g for 10 min. The PIPA scaffolds were placed in petri dishes and vacuum dried for 2 hours (hr) at room temperature before stored at 4°C overnight.
40000 hMSCs were seeded pr scaffold in DMEM high glucose supplemented with 10% FBS, 100 nM dexamethasone, 10 mM β-glycerophosphate, 50 µM L-ascorbic acid-2 phosphate, and 10⁻⁸ M vitamin D and cultured for up to 14 days at 37°C, in a humidified atmosphere of 5% CO₂. Media were changed twice a week.

Gene expression of osteogenic markers were analyzed after 2 hr, 6 hr, 2, 7, and 14 days using RT-qPCR

### Preliminary results

20 and 100 ng/ml BMP2 seem to stimulate the osteogenic transcription factor runx2 after two hours. 100 ng/ml BMP2 seems to stimulate alkaline phosphatase (ALP), an early marker of osteogenic differentiation after two days and 20 ng/ml BMP2 seems to stimulate osteocalcin (OC), a late marker of osteogenic differentiation after 14 days.

### Conclusion

This pilot study indicates that PIPA scaffolds functionalized by 20 and 100 ng/ml BMP2 by dip coating can stimulate osteogenic differentiation of hMSCs. This will be studied further both *in vitro* and *in vivo.*

## Claims

1. A method for modifying a surface morphology of a medical device comprising:
a) providing a medical device comprising a bulk part and a surface region, said surface region comprises a biocompatible polymeric composition comprising a biocompatible polymer;
b) contacting at least a portion of the biocompatible polymeric composition in said surface region with a solvent of the biocompatible polymer to form a first surface region film on at least a first part of said surface region;
c) contacting at least a part of said first surface region film with a non-solvent of said biocompatible polymer, thereby forming a first solidified surface region film on at least a part of said surface region; and
d) subjecting the first solidified surface region film to a hydrolytic treatment.

2. A method according to claim 1, wherein said hydrolytic treatment being performed with an aqueous basic solution.

3. A method according to claim 1 or 2, further comprising the step of:
- sorbing a bioactive agent onto the solidified surface region film.

4. A method according to any one of claims 1-3, wherein the solvent and/or non-solvent of the biocompatible polymer comprise a bioactive agent.

5. A method according to any one of claims 1-4, wherein the bulk part of the medical device comprises a biocompatible polymeric composition identical to the biocompatible polymeric composition in the surface region.

6. A method according to any one of claims 3-5, wherein the bioactive agent is selected from the group consisting of antiviral agents, antimicrobial agents, angiogenic agents, antigenic agents, antitumor agents, immunosuppressants, proteins, peptides, steroids, viral vectors, growth factors, amino acids, polyribonucleic acids, polydeoxyribonucleic acids, chemically modified polynucleic acids, vitamins, hyaluronic acid, bioactive carbohydrates, bioactive lipids bone morphogenic proteins, hormones, enzymes, bone autograft, analgesics, bone marrow, bone allograft, hydroxyapatite, calcium phosphate, calcium carbonate particles, tricalciumphosphate, bioglass, strontium compounds, parenchymal cells, and mesenchymal cells or combinations thereof.

7. A method according to any of the preceding claims, wherein the solid content of the biocompatible polymer in the provided solvent is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%.

8. A method according to any of the preceding claims, wherein the solid content in the provided solvent is in the range 0-10%, such as 0%, such as in the range 0.01-10% (w/w), such as in the range 0.01-5%, such as in the range 0.01-3%, such as in the range 0.01-2%, such as in the range 0.01-1%, such as in the range 0.1-10%, such as in the range 1-10%, such as in the range 2-10%, such as in the range 3-10% or such as in the range 5-10%.

9. The method according to any of the preceding claims, wherein the entire medical device comprises the biocompatible polymeric composition comprising a biocompatible polymer on the surface and wherein the entire medical device is subjected to the method steps b) and c).

10. A medical device obtainable by the method of claims 1-9.

11. A medical device according to claim 10, wherein the medical device is shaped as a framework forming one or more open networks of voids.

12. A medical device according to any one of the claims 10-11, wherein the medical device is selected from the group consisting of bone tissue engineering scaffold, stent, self-expandable stent, balloon expandable stent, stent-graft, graft, aortic graft, heart valve prostheses, cerebrospinal fluid shunt, nerve guide, vascular graft, ureteral graft, urethral graft, pacemaker electrode, catheter, pelvic floor repair membranes, patches, peritoneal patches, endocardial lead, anastomotic device, CABG anastomotic clips and connectors, orthopedic implant, screw, spinal implant, electro-stimulatory device, a bone screw; a cosmetic implant;, and stoma device.

13. A medical device according to any one of the claims 10-12 for use as a medicament.

14. A medical device according to any one of the claims 10-13 for use as a controlled drug release depot.

15. A medical device according to any of claim 10-14, for use as an implantable cell carrier.

## Patentansprüche

1. Verfahren zum Modifizieren einer Oberflächenmorphologie eines medizinischen Produkts, umfassend:
a) Bereitstellen eines medizinischen Produkts, umfassend einen Volumenteil und eine Oberflächenregion, wobei die Oberflächenregion eine biokompatible Polymerzusammensetzung umfasst, umfassend ein biokompatibles Polymer;
b) Kontaktieren mindestens eines Abschnitts der biokompatiblen Polymerzusammensetzung der Oberflächenregion mit einem Lösungsmittel des biokompatiblen Polymers unter Ausbildung eines ersten Oberflächenregionsfilms auf mindestens einem Teil der Oberflächenregion;
c) Kontaktieren mindestens eines Teils des ersten Oberflächenregionsfilms mit einem Nicht-Lösungsmittel des biokompatiblen Polymers, wodurch ein erster verfestigter Oberflächenregionsfilm auf mindestens einem Teil der Oberflächenregion ausgebildet wird; und
d) Unterwerfen des ersten verfestigten Oberflächenregionsfilms einer hydrolytischen Behandlung.

2. Verfahren nach Anspruch 1, wobei die hydrolytische Behandlung mit einer wässrigen alkalischen Lösung durchgeführt wird.

3. Verfahren nach Anspruch 1 oder 2, weiterhin umfassend den Schritt:
- Adsorbieren eines bioaktiven Mittels auf den verfestigten Oberflächenregionsfilm.

4. Verfahren nach einem der Ansprüche 1-3, wobei das Lösungsmittel und/oder das Nicht-Lösungsmittel des biokompatiblen Polymers ein bioaktives Mittel umfasst.

5. Verfahren nach einem der Ansprüche 1-4, wobei der Volumenteil des medizinischen Produkts eine biokompatible Polymerzusammensetzung umfasst, die identisch mit der biokompatiblen Polymerzusammensetzung der Oberflächenregion ist.

6. Verfahren nach einem der Ansprüche 3-5, wobei das bioaktive Mittel ausgewählt ist aus der Gruppe, bestehend aus antiviralen Mitteln, antimikrobiellen Mitteln, angiogenen Mitteln, antigenen Mitteln, Antitumormitteln, Immunsuppressiva, Proteinen, Peptiden, Steroiden, viralen Vektoren, Wachstumsfaktoren, Aminosäuren, Polyribonukleinsäuren, Polydeoxyribonukleinsäuren, chemisch modifizierten Polynukleinsäuren, Vitaminen, Hyaluronsäure, bioaktiven Kohlenwasserstoffen, bioaktiven Lipiden, knochenmorphogenetischen Proteinen, Hormonen, Enzymen, körpereigenem Knochentransplantat, Analgetika, Knochenmark, allogenes Knochentransplantat, Hydroxyapatit, Calciumphosphat, Calciumcarbonat-Partikeln, Tricalciumphosphat, Bioglas, Strontiumverbindungen, Parenchymzellen und Mesenchymzellen oder Kombinationen davon.

7. Verfahren nach einem der vorhergehenden Ansprüche, wobei der Feststoffgehalt des biokompatiblen Polymers in dem bereitgestellten Lösungsmittel im Bereich 0-10 %, wie 0 %, wie im Bereich 0,01-10 Gew.-%, wie im Bereich 0,01-5 %, wie im Bereich 0,01-3 %, wie im Bereich 0,01-2 %, wie im Bereich 0,01-1 %, wie im Bereich 0,1-10 %, wie im Bereich 1-10 %, wie im Bereich 2-10 %, wie im Bereich 3-10 % oder wie im Bereich 5-10 % liegt.

8. Verfahren nach einem der vorhergehenden Ansprüche, wobei der Feststoffgehalt in dem bereitgestellten Lösungsmittel im Bereich 0-10 %, wie 0 %, wie im Bereich 0,01-10 Gew.-%, wie im Bereich 0,01-5 %, wie im Bereich 0,01-3 %, wie im Bereich 0,01-2 %, wie im Bereich 0,01-1 %, wie im Bereich 0,1-10 %, wie im Bereich 1-10 %, wie im Bereich 2-10 %, wie im Bereich 3-10 % oder wie im Bereich 5-10 % liegt.

9. Verfahren nach einem der vorhergehenden Ansprüche, wobei das gesamte medizinische Produkt die biokompatible Polymerzusammensetzung umfassend ein biokompatibles Polymer auf der Oberfläche umfasst und wobei das gesamte medizinische Produkt den Verfahrensschritten b) und c) unterworfen wird.

10. Medizinisches Produkt, das durch das Verfahren nach Anspruch 1-9 erhältlich ist.

11. Medizinisches Produkt nach Anspruch 10, wobei das medizinische Produkt als ein Gestell geformt ist, der sich auf einem oder mehreren offenen Netzwerken aus Poren ausbildet.

12. Medizinisches Produkt nach Anspruch 10-11, wobei das medizinische Produkt ausgewählt ist aus der Gruppe, bestehend aus einem Knochengewebe-Engineering-Gerüst, einem Stent, einem selbstexpandierenden Stent, einem Ballon-expandierenden Stent, einem Stentgraft, einem Transplantat, einem Aortatransplantat, Herzklappenprothesen, einem Zerebrospinalflüssigkeitshunt, einer Nervenleitschiene, einem Gefäßtransplantat, einem Harnleitertransplantat, einem Harnröhrentransplantat, eine Schrittmacherelektrode, einem Katheter, Beckenboden-Reparaturmembranen, Patches, Peritoneal-Patches, einer endokardialen Elektrode, einer Anastomosenvorrichtung, CABG-Anastomosenklammern und -verbindern, einem orthopädischen Implantat, einer Schraube, einem spinalen Implantat, einer elektrostimulierenden Vorrichtung, einer Knochenschraube; einem kosmetischen Implantat und einer Stoma-Vorrichtung.

13. Medizinisches Produkt nach einem der Ansprüche 10-12 zur Verwendung als ein Arzneimittel.

14. Medizinisches Produkt nach einem der Ansprüche 10-13 zur Verwendung als ein Depot zur kontrollierten Wirkstofffreisetzung.

15. Medizinisches Produkt nach einem der Ansprüche 10-14 zur Verwendung als ein implantierbarer Zellträger.

## Revendications

1. Procédé de modification d'une morphologie superficielle d'un dispositif médical, comprenant les étapes suivantes :
a) prévoir un dispositif médical comprenant un corps et une région superficielle, ladite région superficielle comprenant une composition polymère biocompatible comprenant un polymère biocompatible ;
b) mettre en contact au moins une partie de la composition polymère biocompatible de ladite région superficielle avec un solvant du polymère biocompatible pour former un premier film de région superficielle sur au moins une première partie de ladite région superficielle ;
c) mettre en contact au moins une partie dudit premier film de région superficielle avec un agent qui n'est pas un solvant dudit polymère biocompatible, ce qui permet de former un premier film de région superficielle solidifié sur au moins une partie de ladite région superficielle ; et
d) soumettre le premier film de région superficielle solidifié à un traitement hydrolytique.

2. Procédé selon la revendication 1, dans lequel ledit traitement hydrolytique est réalisé au moyen d'une solution de base aqueuse.

3. Procédé selon la revendication 1 ou 2, comprenant en outre l'étape suivante :
- placer un agent bio-actif sorbant sur le film de région superficielle solidifié.

4. Procédé selon l'une quelconque des revendications 1 à 3, dans lequel le solvant et/ou l'agent qui n'est pas un solvant du polymère biocompatible contient un agent bioactif.

5. Procédé selon l'une quelconque des revendications 1 à 4, dans lequel le volume du dispositif médical comprend une composition polymère biocompatible identique à la composition polymère biocompatible de la région superficielle.

6. Procédé selon l'une quelconque des revendications 3 à 5, dans lequel l'agent bioactif est choisi dans le groupe constitué d'agents antiviraux, agents antimicrobiens, agents angiogéniques, agents antigéniques, agents antitumoraux, immunodépresseurs, protéines, peptides, stéroïdes, vecteurs viraux, facteurs de croissance, acides aminés, acides polyribonucléiques, acides polydésoxyribonucléiques, acides polynucléiques modifiés chimiquement, vitamines, acide hyaluronique, hydrates de carbone bioactifs, bioactifs lipides, protéines d'os morphogéniques, hormones, enzymes, autogreffes d'os, analgésiques, moelle osseuse, allogreffes d'os, hydroxyapatite, phosphate de calcium, particules de carbonate de calcium, phosphate tricalcique, bioverre, composés de strontium, cellules parenchymateuses et cellules mésenchymateuses ou des combinaisons de ceux-ci.

7. Procédé selon l'une quelconque des revendications précédentes, dans lequel la teneur en solide du polymère biocompatible dans le solvant prévu est comprise entre 0 et 10 %, par exemple 0 %, par exemple comprise entre 0,01 et 10 % (p/p), par exemple comprise entre 0,01 et 5 %, par exemple comprise entre 0,01 et 3 %, par exemple comprise entre 0,01 et 2 %, par exemple comprise entre 0,01 et 1 %, par exemple comprise entre 0,1 et 10 %, par exemple comprise entre 1 et 10 %, par exemple comprise entre 2 et 10 %, par exemple comprise entre 3 et 10 % ou par exemple comprise entre 5 et 10 %.

8. Procédé selon l'une quelconque des revendications précédentes, dans lequel la teneur en solide dans le solvant prévu est comprise entre 0 et 10 %, par exemple 0 %, par exemple comprise entre 0,1 et 10 % (p/p), par exemple comprise entre 0,01 et 5 %, par exemple comprise entre 0,01 et 3 %, par exemple comprise entre 0,01 et 2 %, par exemple comprise entre 0,01 et 1 %, par exemple comprise entre 0,1 et 10 %, par exemple comprise entre 1 et 10 %, par exemple comprise entre 2 et 10 %, par exemple comprise entre 3 et 10 % ou par exemple comprise entre 5 et 10 %.

9. Procédé selon l'une quelconque des revendications précédentes, dans lequel le dispositif médical entier comprend la composition polymère biocompatible comprenant un polymère biocompatible à la surface et dans lequel le dispositif médical entier est soumis aux étapes b) et c) du procédé.

10. Dispositif médical pouvant être obtenu par le procédé des revendications 1 à 9.

11. Dispositif médical selon la revendication 10, dans lequel le dispositif médical présente une forme de treillis formant au moins un réseau ouvert de vides.

12. Dispositif médical selon l'une quelconque des revendications 10 et 11, dans lequel le dispositif médical est choisi dans le groupe constitué d'un échafaudage d'ingénierie du tissu osseux, un stent, un stent auto-extensible en largeur, un stent extensible à ballon, un stent greffé, une greffe, une greffe de l'aorte, des prothèses de valvules cardiaques, un shunt de fluide cérébrospinal, un tube guide nerveux, une greffe vasculaire, une greffe urétérale, une greffe urétrale, une électrode de stimulation cardiaque, un cathéter, des membranes de réparation du plancher pelvien, des greffes en pièce, des greffes péritonéales en pièce, un fil endocardiaque, un dispositif anastomotique, des pinces et connecteurs anastomotiques de pontage aortocoronarien, un implant orthopédique, une vis, un implant spinal, un dispositif de stimulation électrique, une vis à os ; un implant cosmétique ; et un dispositif stomatique.

13. Dispositif médical selon l'une quelconque des revendications 10 à 12, destiné à être utilisé comme médicament.

14. Dispositif médical selon l'une quelconque des revendications 10 à 13, destiné à être utilisé comme dépôt de médicament contrôlé.

15. Dispositif médical selon l'une quelconque des revendications 10 à 14, destiné à être utilisé comme porteur de cellule implantable.
